# EUROPEAN PATENT SPECIFICATION

(11) **EP 2 580 343 B1**
(45) Date of publication and mention of the grant of the patent: **05.10.2016**
(21) Application number: 11770820.6
(22) Date of filing: 13.06.2011
(51) Int. Cl.: C12Q 1/04, A61K 35/74, C12Q 1/68, A61K 35/741

(54) **USE OF ABO TYPE IN A METHOD FOR TAILORING A BACTERIAL COMPOSITION**
VERWENDUNG VON ABO TYP IN EINEM HERSTELLUNGSVERFHAREN EINER BEDARFSGERECHTEN BAKTERIELLEN ZUSAMMENSETZUNG
UTILISATION DU TYPE ABO DANS UNE METHODE DE PRODUCTION D'UNE COMPOSITION BACTERIENNE SUR MESURE

(30) Priority: 11.06.2010 FI 20105670
(43) Date of publication of application: 17.04.2013
(73) Proprietor: DuPont Nutrition Biosciences ApS, 1411 Copenhagen K (DK)
(72) Inventor: MÄKIVUOKKO, Harri, FI-02400 Kirkkonummi (FI); MÄTTÖ, Jaana, FI-00310 Helsinki (FI); WACKLIN, Pirjo, FI-00310 Helsinki (FI); PARTANEN, Jukka, FI-00310 Helsinki (FI); RÄBINÄ, Jarkko, FI-00310 Helsinki (FI)
(74) Representative: D Young & Co LLP
(86) International application number: PCT/FI2011/050558
(87) International publication number: WO 2011/154616

(56) References cited:
- WO-A1-02/070670
- WO-A2-2008/134450
- US-A1- 2008 160 565
- US-A1- 2009 214 497
- US-A1- 2010 120 106
- UCHIDA H ET AL: "A new assay using surface plasmon resonance (SPR) to determine binding of the Lactobacillus acidophilus group to human colonic mucin", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 68, no. 5, 23 May 2004 (2004-05-23), pages 1004-1010, XP002999588, ISSN: 0916-8451, DOI: 10.1271/BBB.68.1004
- HIDEAKI UCHIDA ET AL: "Lactic Acid Bacteria (LAB) Bind to Human B- or H-Antigens Expressed on Intestinal Mucosa", BIOSCIENCE, BIOTECHNOLOGY, AND BIOCHEMISTRY, vol. 70, no. 12, 1 December 2006 (2006-12-01), pages 3073-3076, XP55015057, ISSN: 0916-8451, DOI: 10.1271/bbb.60407
- THEUNISSEN J ET AL: "Identification of probiotic microorganisms in South African products using PCR-based DGGE analysis", INTERNATIONAL JOURNAL OF FOOD MICROBIOLOGY, ELSEVIER BV, NL, vol. 98, no. 1, 15 January 2005 (2005-01-15), pages 11-21, XP027663216, ISSN: 0168-1605 [retrieved on 2005-01-15]
- ROOS S ET AL: "LACTOBACILLUS MUCOSAE SP. NOV., A NEW SPECIES WITH IN VITRO MUCUS-BINDING ACTIVITY ISOLATED FROM PIG INTESTINE", INTERNATIONAL JOURNAL OF SYSTEMATIC AND EVOLUTIONARY MICROBIOLOGY, SOCIETY FOR GENERAL MICROBIOLOGY, READING, GB, vol. 50, no. 6 part 1, 1 January 2000 (2000-01-01), pages 251-258, XP001041591, ISSN: 1466-5026
- M. JOSÉ POZUELO DE FELIPE, ET AL: "Monitoring of the succession of bacterial populations in inant faeces by PCR-denaturing gradient gel electrophoresis", MICROBIAL ECOLOGY IN HEALTH AND DISEASE, vol. 17, 2005, pages 205-211,
- H. G.H.J. Heilig ET AL: "Molecular Diversity of Lactobacillus spp. and Other Lactic Acid Bacteria in the Human Intestine as Determined by Specific Amplification of 16S Ribosomal DNA", Applied and Environmental Microbiology, vol. 68, no. 1, 1 January 2002 (2002-01-01), pages 114-123, XP055159977, ISSN: 0099-2240, DOI: 10.1128/AEM.68.1.114-123.2002

### SEQUENCE LISTING

<110> Suomen Punainen Risti Veripalvelu
<120> Use of ABO type
<130> 2100800PC
<160> 10
<170> PatentIn version 3.5
<210> 1
   <211> 308
   <212> DNA
   <213> Lactobacillus
<400> 1
   caatggacga aagtctgatg gagcaacgcc gcgtgagtga agaagggttt cggctcgtaa      60
   aactctgttg ttaaagaaga acatatctga gagtaactgt tcaggtattg acggtattta     120
   accagaaagc cacggctaac tacgtgccag cagccgcggt aatacgtagg tggcaagcgt     180
   tgtccggatt tattgggcgt aaagcgagcg caggcggttt tttaagtctg atgtgaaagc     240
   cttcggctca accgaagaag tgcatcggaa actgggaaac ttgagtgcag aagaggacag     300
   tggaactc                                                              308
<210> 2
   <211> 260
   <212> DNA
   <213> Lactobacillus
<400> 2
   ttcggatcgt aaaactctgt tgttggagaa gaatgtatct gatagtaact gatcaggtag      60
   tgacggtatc caaccagaaa gccacggcta actacgtgcc agcagccgcg gtaatacgta     120
   ggtggcaagc gttgtccgga tttattgggc gtaaagcgag cgcaggcggt ttcttaagtc     180
   tgatgtgaaa gccttcggct caaccgaaga agtgcatcgg aaactgggaa acttgagtgc     240
   agaagaggac agtggaactc                                                 260
<210> 3
   <211> 308
   <212> DNA
   <213> Lactobacillus
<400> 3
   caatggacgc aagtctgatg gagcaacgcc gcgtgagtga agaaggcttt cgggtcgtaa      60
   aactctgttg ttggagaaga atggtcggca gagtaactgt tgtcggcgtg acggtatcca     120
   accagaaagc cacggctaac tacgtgccag cagccgcggt aatacgtagg tggcaagcgt     180
   tatccggatt tattgggcgt aaagcgagcg caggcggttt tttaagtctg atgtgaaagc     240
   cctcggctta accgaggaag cgcatcggaa actgggaaac ttgagtgcag aagaggacag     300
   tggaactc                                                              308
<210> 4
   <211> 308
   <212> DNA
   <213> Lactobacillus
<400> 4
   caatgggcgc aagcctgatg gagcaacacc gcgtgagtga agaagggttt cggctcgtaa     60
   agctctgttg ttagagaaga acgtgcgtga gagcaactgt tcacgcagtg acggtatcta    120
   accagaaagt cacggctaac tacgtgccag cagccgcggt aatacgtagg tggcaagcgt    180
   tatccggatt tattgggcgt aaagcgagcg caggcggttt gataagtctg atgtgaaagc    240
   ctttggctta accaaagaag tgcatcggaa actgtcagac ttgagtgcag aagaggacag    300
   tggaactc                                                             308
<210> 5
   <211> 308
   <212> DNA
   <213> Lactobacillus
<400> 5
   caatggacgc aagtctgatg gagcaacgcc gcgtgagtga agaaggcttt cgggtcgtaa     60
   aactctgttg ttggagaaga atggtcggca gagtaactgt tgtcggcgtg acggtatcca    120
   accagaaagc cacggctaac tacgtgccag cagccgcggt aatacgtagg tggcaagcgt    180
   tatccggatt tattgggcgt aaagcgagcg caggcggttt tttaagtctg atgtgaaagc    240
   cctcggctta accgaggaag tgcatcggaa actgggaaac ttgagtgcag aagaggacag    300
   tggaactc                                                             308
<210> 6
   <211> 307
   <212> DNA
   <213> Lactobacillus
<400> 6
   aatggacgca agtctgatgg agcaacgccg cgtgagtgaa gaaggctttc gggtcgtaaa     60
   actctgttgt tggagaagaa tggtcggcag agtaactgtt gtcggcgtga cggtatccaa    120
   ccagaaagcc acggctaact acgtgccagc agccgcggta atacgtaggt ggcaagcgtt    180
   atccggattt attgggcgta aagcgagcgc aggcggtttt ttaagtctga tgtgaaagcc    240
   ctcggcttaa ccgaggaagy gcatcggaaa ctgggaaact tgagtgcaga agaggacagt    300
   ggaactc                                                              307
<210> 7
   <211> 250
   <212> DNA
   <213> Lactobacillus
<400> 7
   caatggacgc aagtctgatg gagcaacgcc gcgtgagtga agaagggttt cggctcgtaa 60
   agctctgttg gtagtgaaga aagatagagg tagtaactgg cctttatttg acggtaatta 120
   cttagaaagt cacggctaac tacgtgccag cagccgcggt aatacgtagg tggcaagcgt 180
   tgtccggatt tattgggcgt aaagcgagtg caggcggttc aataagtctg atgtgaaagc 240
   cttcggctca 250
<210> 8
   <211> 212
   <212> DNA
   <213> Lactobacillus
<400> 8
   caatgggcgc aagcctgatg gagcaacacc gcgtgagtga agaagggttt cggctcgtaa 60
   agctctgttg ttggagaaga acgtgcgtga gagtaactgt tcacgcagtg acggtatcca 120
   accagaaagt cacggctaac tacgtgccag cagccgcggt aatacgtagg tggcaagcgt 180
   tatccggatt tattgggcgt aaagcgagcg ca 212
<210> 9
   <211> 255
   <212> DNA
   <213> Bifidobacterium
<400> 9
   ctccagttgg atgcatgtcc ttctgggaaa gattcatcgg tatgggatgg ggtcgcgtcc 60
   tatcagcttg atggcggggt aacggcccac catggcttcg acgggtagcc ggcctgagag 120
   ggcgaccggc cacattggga ctgagatacg gcccagactc ctacgggagg cagcagtggg 180
   gaatattgca caatgggcgc aagcctgatg cagcgacgcc gcgtgcggga tgacggcctt 240
   cgggttgtaa accgc 255
<210> 10
   <211> 255
   <212> DNA
   <213> Bifidobacterium
<400> 10
   ctccagttga tcgcatggtc ttctgggaaa gctttcgcgg tatgggatgg ggtcgcgtcc 60
   tatcagcttg acggcggggt aacggcccac cgtggcttcg acgggtagcc ggcctgagag 120
   ggcgaccggc cacattggga ctgagatacg gcccagactc ctacgggagg cagcagtggg 180
   gaatattgca caatgggcgc aagcctgatg cagcgacgcc gcgtgaggga tggaggcctt 240
   cgggttgtaa acctc 255

## Description

### Field of the invention

The present invention relates to a method of tailoring a bacterial composition for an individual having certainABO blood group genotype based on the differences in the spectrum of bacteria found between individuals with different ABO blood group status.

### Background of the invention

The human gastrointestinal tract (GIT) comprises an extremely dense and diverse microbiota. The GIT of an adult may harbour even 2 kg of bacterial biomass representing over 1 000 bacterial species, of which majority cannot be cultivated (Eckburg PB et al., Science 2005; 308:1635-8). This microbiota - the term "microbiota" refering to the entire repertoire of microbes found or residing in any certain tissue - in the large intestine is mainly composed of Firmicutes and Bacteroides phyla, which make up over 75% and 16% of total microbes in the GIT (Eckburg PB, et al., 2005). In spite of low diversity at the microbial phyla level, the gut microbiota composition among individuals is highly variable at species and strain level. The microbiota of healthy adult is stable and unique within an individual. The similarity of the dominant microbial population is higher in monozygotic twins compared to dizygotic twins (Zoetendal EG, et al., Syst Appl Microbiol. 2001;24:405-10), suggesting the role of host genetic factors on the microbiota composition. However, little is known in more detailed level which genes or other factors determine or regulate the spectrum of microbial composition.

The microbial biomass in the large intestine is mainly residing in the lumen and the mucosa-associated population differs from the lumen population (Eckburg PB et al., 2005). Intestinal mucus forms a physical barrier between the lumen and the epithelial cells. Although the mucus layer prevents the direct contact of the bacteria with the epithelial cells, it provides adhesion sites for the GIT bacteria and has thus an important role in bacterial colonization. The pathogenic microbe *Helicobacter pylori* and some viruses have shown to use ABO blood group antigens as adhesion reseptors (Boren et al. Science 1993, 262, 1892-1895). Besides adhesion sites, secreted mucus provides endogenous substrate for bacteria. The mucus may be a major nutrient source in situations, where carbohydrates originating elsewhere are limited (Bäckhed F et al., Science. 2005; 307:1915-20.) Some microbes e.g. Bifidobacteria and *Bacteroides thetaiotaomicron* are also able to specifically utilize blood group antigens, e.g. the glycan structures of ABO antigens. There is a continuous interplay between the mucus secretion and degradation by bacteria as bacterial metabolites have been shown to act as signalling molecules modulating the mucus synthesis (Freitas M et al., Biol Cell. 2003; 95: 503-6). The mucus is mainly composed of mucins, large glycoproteins containing a protein core and attached oligosaccharides (Shirazi T et al., Postgrad Med J. 2000; 76: 473-8.).

The gut microbiota has a role in human health. It contributes to the maturation of the gut tissue, to host nutrition, pathogen resistance, epithelial cell proliferation, host energy metabolism and immune response (e.g. Dethlefsen L et al., Trends Ecol Evol. 2006; 21: 517-23; Round JL and Mazmanian SK, Nat Rev Immunol. 2009; 9: 313-23). An altered composition and diversity of gut microbiota have been associated to several diseases (Round JL and Mazmanian SK, 2009), such as inflammatory bowel disease, IBD (Sokol H et al., World J Gastroenterol. 2008; 14: 3497-503), irritable bowel syndrome (Mättö J et al., FEMS Immunol Med Microbiol. 2005; 43: 213-22), rheumatoid arthritis (Vaahtovuo J et al., J Rheumatol. 2008; 35: 1500-5.), atopic eczema (Kalliomäki M et al., Lancet. 2003 ;361: 1869-71), asthma (Björkstén B, Proc Nutr Soc. 1999; 58: 729-32) and type 1 diabetes (Wen L et al., Nature, 2008; 455: 1109-13). The beneficial effects of certain microbial species or strains on maintaining or even improving the gut balance and growing evidence of their health effects on intestinal disturbances have lead to a great interest on modulation of gut microbiota. Gut microbiota can be modulated by probiotics; the majority currently belonging to the *Bifidobacterium* and *Lactobacillus* genera.

In addition to the microbiota of the gut, other mucosal tissues, such as uro-genital tract, skin, oral or nasal tissues, have their own repertoire of commensal microbes. The balance of the microbiota in these tissues similarly is important to the well-being of the host. Not much is known yet of the spectrum of microbes in these tissues. In healthy vagina several species of *Lactobacillus* spp. including, *L. crispatus, L. gasseri, L. jensenii* and *L. iners* predominate. In bacterial vaginitis the balance of the microbiota is shifted towards colonisation by anaerobes; especially *Gardnerella vaginalis, Atopobium* spp., several *Prevotella* spp. e.g. *P. bivia* and *P. buccalis, Megaspaera* spp. may be commonly detected (Srinivasan S and Fredricks DN. Interdiscip Perspect Infect Dis. 2008; 2008: 750479). Similarly to the intestine, the diversity of oral microbiota is enormous. Healthy oral cavity is colonised mainly by facultative gram positive cocci and rods, e.g. *Streptococcus spp.* and *Actinomyces* spp. predominate in healthy oral cavity. During gingival inflammation the microbiota tends to change towards predominance of gram-negative anaerobes e.g. *Prevotella* spp. and *Veillonella* spp (Ledder RG et al., Appl Environ Microbiol. 2007; 73: 516-23). A specific oral pathogen, *Streptococcus mutans* is associated with dental caries and several putative pathogens e.g. *Porphyromonas gingivalis, Aggregatibacterium actinomyce-temcomitans* and *Prevotella intermedia* are associated with periodontal diseases. In addition, mucosal sites can be colonised by *Candida* spp. which may lead to candidiasis. In all these mucosal sites the microbiota is highly individual but it is not known which factors determine the spectrum.

Probiotic supplements and microbiota modulation products currently on the market are ineffective in promoting the desired health effects among most individuals. Thus, there is a continuous need for more specific or personally tailored products that are able to mediate the health effects of the microbes more efficiently.

### Brief description of the invention

The present invention is based on the finding that the ABO blood group status of the host determines surprisingly strongly the repertoire of the bacterial strains or species found in the gut - the site where the vast majority of commensal microbes reside. Hence, ABO blood group is a major genetic determinant for the composition of the host microbes of mucosal tissues. Using DGGE analyses and DNA sequencing of the corresponding 16S ribosomal RNAs, the present invention demonstrated that certain microbial genotypes were strongly specific to, or were found to prefer individuals with certain ABO status. Similarly, the analysis of the G+C contents of bacterial DNAs in samples from individuals grouped according to their ABO blood group supported the finding. This invention can be utilised to prepare a microbial and/or probiotic composition targeted to specific consumer or recipient groups, and/or to prepare a personally-tailored microbial and/or probiotic composition, based on the microbial genotypes that were found to be specific to or to prefer certain ABO blood groups of an individual. The tailored composition increases the efficacy and/or potency of the microbial composition, as it can be modulated so that microbial strains and/or genotypes are those that preferentially survive at and colonise the gut and/or other mucosal sites of an individual. As some pathogenic microbes are known to utilize the ABO molecules, the invention can be applied to prepare products efficiently competing with the pathogenic microbes. Hence, microbial and/or probiotic compositions and products, and/or other types of microbiota modulations can be tailored based on the ABO status of an individual.

Thus, an object of the present invention is
a method of tailoring a microbial
composition for an individual having certain ABO blood group genotype based on the spectrum of bacteria found in the mucous tissue of at least one individual with the same ABO blood group genotype, comprising:
determining the ABO blood type of the recipient of the bacterial composition;
determining the typical mucosal bacterial repertoire of the specific ABO blood type by determining which bacterial strains are more common in the intestine of individuals having:
   (a) B or AB blood group genotypes than individuals having A or O blood group genotypes;
   (b) A or AB blood group genotypes than individuals having B or O blood group genotypes; and/or
   (c) A, B or AB blood group genotypes than individuals having O blood group genotype; and
manufacturing the bacterial composition based on the determined bacterial repertoire.

The object of the invention is achieved by the method set forth in the independent claim. Preferred embodiments of the invention are described in the dependent claims.

Other objects, details and advantages of the present invention will become apparent from the following drawings, detailed description and examples.

### Brief description of the drawings

Figure 1 shows the %G+C-profiles of the samples grouped and averaged
   by ABO blood group status.
Figure 2 shows the average volatile fatty acid profile (SCFA) and lactic acid concentrations with ±SD of samples grouped by ABO blood group status. Statistical significances were tested with Student's t-test; no significant differences were found.

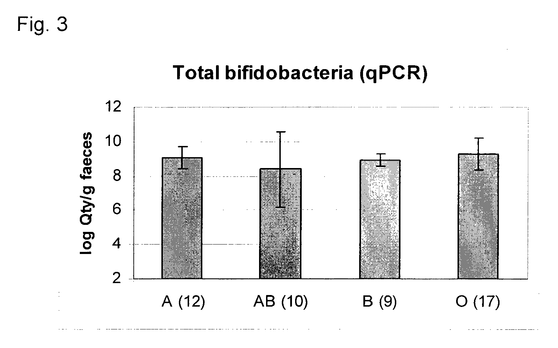

Figure 3 shows the qPCR-analysis of bifidobacteria genus with ±SD of samples grouped by ABO blood group status. Statistical significances were tested with Student's t-test; no significant differences were found. Figure 4 illustrates the clustering of the samples in PCA analysis of the PCR-DGGE profiles obtained with universal bacterial primers in Example 3.
Figure 5 illustrates the diversity of faecal microbiota in samples of individuals grouped according to their A, AB, B and 0 blood group in Example 3. Shannon diversity index calculated from intensity matrix of the PCR-DGGE profiles obtained with universal bacterial primers.
Figure 6 illustrates the clustering of the samples in PCA analysis of the PCR-DGGE profiles obtained with EREC-group specific bacterial primers in Example 4.
Figure 7 illustrates the diversity of faecal microbiota in samples of individuals grouped according to their A, AB, B and O blood group in example 4. Shannon diversity index based on intensity matrix of PCR-DGGE profiles obtained with EREC-group specific primers.
Figure 8 illustrates the clustering of the samples in PCA analysis of the PCR-DGGE profiles obtained with *B. fragilis* -group specific bacterial primers in Example 5.
Figure 9 illustrates the diversity of faecal microbiota in Example 5. Shannon diversity index in the PCR-DGGE profiles obtained with *B. fragilis -* group specific primers in samples of individuals grouped according to their A, AB, B and O blood group.
Figure 10 illustrates the clustering of the samples in PCA analysis of the PCR-DGGE profiles obtained with C. *leptum* -group specific bacterial primers in Example 6.
Figure 11 illustrates the diversity of faecal microbiota in Example 6. Shannon diversity index in the PCR-DGGE profiles obtained with C. *leptum* -group specific primers in samples of individuals grouped according to their A, AB, B and O blood group.
Figure 12 illustrates an example of PCR-DGGE profiles obtained with Lactobacillus-group specific primers.
Figure 13 illustrates the clustering of the samples in PCA analysis of the PCR-DGGE profiles obtained with *Lactobacillus* -group specific bacterial primers in Example 7.
Figure 14 illustrates the diversity of faecal microbiota in Example 7. Shannon diversity index in the PCR-DGGE profiles obtained with *Lactobacillus* -group specific primers in samples of individuals grouped according to their A, AB, B and O blood group.
Figure 15 illustrates the clustering of the samples in PCA analysis of the PCR-DGGE profiles obtained with *Bifidobacterium* -group specific bacterial primers in Example 8.
Figure 16 illustrates the diversity of faecal microbiota in Example 8. Shannon diversity index in the PCR-DGGE profiles obtained with *Bifidobacterium* -group specific primers in samples of individuals grouped according to their A, AB, B and O blood group.
Figure 17 illustrates the clustering of the samples in PCA analysis of the PCR-DGGE profiles obtained with different primers using ABO blood group status as a grouping factor in Example 10. A: Universal group, B: EREC group, C: BFRA (*B. fragilis)* group, D: *C.leptum* group, E: *Lactobacillus* group, F: *Bifidobacterium* group.
Figure 18 illustrates the clustering of the samples in RDA analysis of the PCR-DGGE profiles obtained with universal bacterial primers using ABO blood group status as a grouping factor in Example 10.
Figure 19 illustrates the Shannon Diversity index calculations of the PCR-DGGE profiles obtained with universal bacterial primers using ABO blood group status as a grouping factor in Example 10. Columns are averaged values of the corresponding ABO blood data and ±SD is marked on each column. Trend-like difference based on two-tailed t-tests between A and B blood groups is marked with a diagonal bar and with the corresponding p-value.
Figure 20 illustrates the clustering of the samples in RDA analysis of the PCR-DGGE profiles obtained with EREC primers using ABO blood group status as a grouping factor in Example 11.
Figure 21 illustrates the Shannon Diversity index calculations of the PCR-DGGE profiles obtained with EREC primers using ABO blood group status as a grouping factor in Example 11. Columns are averaged values of the corresponding ABO blood data and ±SD is marked on each column. Statistically significant differences based on two-tailed t-tests between ABO blood groups are marked with diagonal bars and with the corresponding p-value.
Figure 22 illustrates the clustering of the samples in RDA analysis of the PCR-DGGE profiles obtained with BFRA primers using ABO blood group status as a grouping factor in Example 12.
Figure 23 illustrates the Shannon Diversity index calculations of the PCR-DGGE profiles obtained with BFRA primers using ABO blood group status as a grouping factor in Example 12. Columns are averaged values of the corresponding ABO blood data and ±SD is marked on each column.
Figure 24 illustrates the clustering of the samples in RDA analysis of the PCR-DGGE profiles obtained with C. *leptum* primers using ABO blood group status as a grouping factor in Example 13.
Figure 25 illustrates the Shannon Diversity index calculations of the PCR-DGGE profiles obtained with C. *leptum* primers using ABO blood group status as a grouping factor in Example 13. Columns are averaged values of the corresponding ABO blood data and ±SD is marked on each column. Statistically significant differences based on two-tailed t-tests between ABO blood groups are marked with diagonal bars and with the corresponding p-value.
Figure 26 illustrates the clustering of the samples in RDA analysis of the PCR-DGGE profiles obtained with *Lactobacillus* primers using ABO blood group status as a grouping factor in Example 14.
Figure 27 illustrates the Shannon Diversity index calculations of the PCR-DGGE profiles obtained with *Lactobacillus* primers using ABO blood group status as a grouping factor in Example 14. Columns are averaged values of the corresponding ABO blood data and ±SD is marked on each column.
Figure 28 illustrates the clustering of the samples in RDA analysis of the PCR-DGGE profiles obtained with *Bifidobacterium* primers using ABO blood group status as a grouping factor in Example 15.
Figure 29 illustrates the Shannon Diversity index calculations of the PCR-DGGE profiles obtained with *Bifidobacterium* primers using ABO blood group status as a grouping factor in Example 15. Columns are averaged values of the corresponding ABO blood data and ±SD is marked on each column. Statistically significant differences based on two-tailed t-tests between ABO blood groups are marked with diagonal bars and with the corresponding p-value.
Figure 30 illustrates the clustering of the samples in RDA analysis of the PCR-DGGE profiles obtained using the presence or absence of B-antigen as a grouping factor. Universal primer data in Panel A, C. *leptum* primer data in Panel B and EREC primer data in Panel C. Non-B-antigen blood group data points are marked red and B-antigen blood group data points blue.
Figure 31 shows the adhesion efficiency of bacterial strains (*L. cris-patus* LMG 18199, *L. rhamnosus* LGG, *L. casei* ATCC 334, *B. animalis* subsp. *lactis* Bb-12) to blood group antigens in an *in vitro* adhesion test. LMG column is the average figure of 40 independent tests and LGG columns are the average figures of 8 independent tests.

### Detailed description of the invention

Mucosal tissues such as gastrointestinal tract, oral cavity and vagina are colonized by microbes which are considered as essential for maintaining and/or promoting health of an individual. High microbial diversity in the gut, for example, is beneficial for the health of an individual, because the bacteria can, for example, prevent adhesion of adverse microbes on gut epithelium and prevent their colonisation in the intestine. They may also modulate the immune response of the host.

The present invention is based on the finding that the individuals with different ABO blood group genotypes have differences in the repertoire of microbes in their intestinal bacterial population. Further, the present invention is based on the finding that the individuals with different ABO blood group genotypes have differences in the diversity of microbes in their intestinal microbial population. In particular, the repertoire of microbes in individuals with B -antigen was differently clustered from that in non-B-individuals, as demonstrated by using statistical approaches. The clustering effect is particularly strong in Universal, EREC and *C. leptum* PCR-DGGE groups, where individuals having the B-antigen molecules in their gastrointestinal secretions clearly differ from non-B-antigen individuals. This result indicates that persons with blood group B or AB have different bacterial compositions in the most abundant gastrointestinal microbe groups compared to persons with blood group A or O. As the microbial species corresponding to each DGGE genotype can be identified e.g. by sequencing, a detailed repertoire of specific microbes for each individual and a typical repertoire for individuals with a specific ABO type can be determined.

These findings can be used as a basis for targeted modulation of the microbial population in the individuals with different ABO blood group genotypes and as a criterion for personally tailored microbial and/or probiotic supplementation. As some pathogenic microbes are known to utilize the ABO molecules, the invention can be applied to prepare products efficiently competing with the pathogenic microbes.

In the present invention, the use of method "percent guanidine-cytosine (%G+C) profiling" revealed several shifts in overall microbiota composition, representing the predominant microbiota between subjects from different blood groups. These differences in microbiota indicated that individuals with different ABO blood groups had different microbiota composition.

The genomic DNA in microbe samples from individuals were profiled using the %G+C-profiling technique, which allows the identification of microbial clusters or subsets, including also unculturable microbes, according to their genomic G+C contents, in samples. The method is well described in the art (Apajalahti JH, et al., Appl Environ Microbiol. 1998; 64: 4084-8; Vanhoutte et al. FEMS Microb Ecol 2004; 48; 437-446; Matsuki et al. Applied and Environmental Microbiology 2004; 70; 7220-7228; Satokari et al. AEM 2001; 67: 504-513; Mättö et al. FEMS Immunol Med Microbiol. 2005; 43: 213-22). The method is based on molecular weight difference between A-T and G-C linkages in DNA double helix. DNA strands rich in G-C residues are heavier than A-T rich strands. The strands can be separated in a salt gradient by ultra speed centrifugation. The sample tubes are emptied by pumping out the content of the tube. As the DNA strands are tagged with fluorescent dye, the dye intensity can be recorded and the G+C content calculated.

In the present invention, it was found that Denaturating Gradient Gel Electrophoresis (DGGE) genotypes:
UNIVERSAL (UNIV) DGGE genotypes: 18.00%, 18.40%, 31.40%, 32.20%, 42.20% 47.00%, 58.80%, 61.10%;
EREC DGGE genotypes: 35.30%, 39.70%, 46.90%, 50.90%, 61.10%, 73.90%;
C. *leptum* DGGE genotypes: 15.40%, 16.00%, 20.50%, 38.80%, 67.90%;
Lacto 9.00%; and
Bfra DGGE genotypes 5.00%, 21.50%, 25.90% and 41.10%, were associated with the presence of blood group B antigen.

Especially, it was found that DGGE genotypes:
UNIVERSAL DGGE genotypes: 18.00%, 31.40%, 32.20%, 42.20% 47.00%, 58.80%, 61.10%;
EREC DGGE genotypes: 35.30%, 39.70%, 46.90%, 50.90%, 61.10%, 73.90%;
C. *leptum* DGGE genotypes: 15.40%, 16.00%, 20.50%, 38.80%, 67.90%; and
Bfra DGGE genotypes 5.00%, 21.50% were significantly associated with the presence of blood group B antigen.

In addition, genotypes UNIV 39.00%, UNIV 31.20%, EREC 4.8% and Bfra 9.90% were associated with the presence of the blood group A antigen. Further, genotypes UNIV 39.00%, EREC 4.8% and Bfra 9.90% were significantly associated with the presence of the blood group A antigen.

Genotypes Lacto 74.20%, 86.60%, 92.30%, C. *leptum* 52.10%, 84.00%, Bfra 62.80% and Bifido 26.60% were significantly associated with the presence of the blood group O antigen.

Certain genotypes were significantly associated with the absence of a particular ABO antigen, rather than the presence; examples were UNIV 49.40% and Bfra 36.80% that were not found in the B positive samples, and Lacto 14.10%, EREC 39.70% and EREC 46.90% that were rare in blood group O positives.

The *Bacteroides* population as a whole was significantly more diverse in the B and AB blood groups, i.e. individual positive for the B antigen, than in A and O blood groups (non-B individuals). This finding shows a need and creates a potential for targeted microbiota modulation according to host's ABO blood group. It can be applied to prevent the overgrowth of Bacteroidetes in various mucosal sites. Bacteroidetes comprise a predominant part of the microbiota in the intestine, additionally bacteria of this group, in particular *Prevotella* and *Porphyromonas* spp., have been detected in increased incidence and numbers in other mucosal diseases. Similarly, dominant microbiota (UNIV DGGE), and C. *leptum* group microbiota were significantly more diverse in the B blood group in comparison to other blood groups (UNIV B vs A, C. leptum B vs AB, A or O). Moreover, RDA analysis showed a clear B-antigen specific clustering of the dominant (UNIV DGGE), EREC group and C. *leptum* group microbiota, in the non-B antigen samples. This finding shows a need and creates the potential for the consideration of the ABO blood group genotype of an individual in applications related to the microbiota modulation.

Denaturating Gradient Gel Electrophoresis, DGGE, is a method of choice to detect differences in spectrum or abundance of different bacterial genotypes. The method is well described in the art (Vanhoutte et al. FEMS Microb Ecol 2004; 48; 437-446; Matsuki et al. Applied and Environmental Microbiology 2004; 70; 7220-7228; Satokari et al. AEM 2001; 67: 504-513; Mättö et al. FEMS Immunol Med Microbiol. 2005; 43: 213-22). In the method, specific PCR primers are designed so that in each experimental setting, only the desired bacterial group or groups are analysed, hence e.g. term "Lactobacillus DGGE" in which primers specific for Lactobacillus strains are used (Table 1). The differences in band positions and/or their occurrence and/or intensity indicate differences in bacterial compositions between faecal samples. Base composition of the PCR amplified fragment determinates the melting and, thus, the mobility of the fragment in the denaturing gradient in gel. The final position of the fragment in gel is consequently specified by the DNA sequence of the fragment, the applied denaturing gradient and the electrophoresis running conditions. The optimised running conditions and denaturing gradient of the gels for the bacterial groups used in this invention are shown in Table 2. The position of each fragment, the "band position", between different gel runs are normalised by using standards. The band position is indicated relative to length of the gel, the top being 0% and the bottom edge being 100%. The standards used were composed of PCR amplified fragments of the relevant strains belonging to each bacterial group as described in Table 2.

The term "genotype" refers to those strains having the essentially same band position in the relevant DGGE analysis. Each genotype or a group of closely-related genotypes can be presented as a band position. In the present invention, each band position refers to the band positions of the given %-value +/- 1% unit, i.e. 25.30% refers to any value between 24.30% and 26.30%, when analysed using the methodology described above. It is noted that depending on the exact conditions the nominal %-value can vary; the relative position of the band to the relevant standard is important.

In the present invention, we identified the lactobacilli and bifidobacteria compositions in more detail with the 16S rRNA nucleotide sequence for each band position. Briefly, we excised the band positions (= DNA fragments) from DGGE gels showing the profiles of faecal samples and sequenced the DNA fragments in the bands. The 16S rRNA fragment sequences of the DGGE genotypes detected are shown in Tables 17 and 18 of Example 17.

In one embodiment, the microbial composition tailored for those with blood group O comprises or is enriched with at least one of the strains with DGGE genotype Lacto 74.20%, 86.60%, 92.30%, C. *leptum* 52.10%, 84.00%, Bfra 62.80%, and/or Bifido 26.60%. In another embodiment, the microbial composition tailored for those with blood group O comprises or is enriched with at least one bacterial strain having DGGE genotype Lacto 92.3%, 86.6% and/or 74.2%. In another embodiment, the microbial composition tailored for those with blood group O comprises or is enriched with DGGE genotype Lacto 92.30% together with DGGE genotype Lacto 86.60% or DGGE genotype Lacto 74.20%, or with both. In another embodiment, the microbial composition tailored for those with blood group O comprises or is enriched with at least one bacterial strain having DGGE genotype Lacto 92.3% and DNA sequence encoding the 16S rRNA identified as SEQ ID NO: 8, DGGE genotype Lacto 86.6% and DNA sequence encoding the 16S rRNA identified as SEQ ID NO: 7 and/or DGGE genotype Lacto 74.20% and DNA sequence encoding the 16S rRNA identified as SEQ ID NO: 4. In a further embodiment, the microbial composition tailored for those with blood group O comprises or is enriched with bacterial strains having DGGE genotype Lacto 92.3% and DNA sequence encoding the 16S rRNA identified as SEQ ID NO:8 together with DGGE genotype Lacto 86.60% and DNA sequence encoding the 16S rRNA identified as SEQ ID NO: 7 or with DGGE genotype Lacto 74.20% and DNA sequence encoding the 16S rRNA identified as SEQ ID NO:4, or with both.

In one embodiment, the microbial composition tailored for those with blood group B and/or AB, i.e, antigen B positives, comprises or is enriched with at least one of the strains having DGGE genotypes UNIVERSAL: 18.00%, 31.40%, 32.20%, 42.20% 47.00%, 58.80%, 61.10%; EREC: 35.30%, 39.70%, 46.90%, 50.90%, 61.10%, 73,90%; C. *leptum:* 15.40%, 16.00%, 20.50%, 38.80%, 67.90%; and Bfra: 5.00%, 21.50%.

In one embodiment, the microbial composition tailored for those with blood group A and/or AB, i.e, antigen A positives, comprises or is enriched with at least one of the strains having DGGE genotype UNIV 31.20%,UNIV 39.00%, EREC 4.8% and Bfra 9.90%. In another embodiment the microbial composition tailored for those with blood group A and/or AB, i.e, antigen A positives, comprises or is enriched with at least one of the strains having DGGE genotype UNIV 39.00%, EREC 4.8% and Bfra 9.90%.

In another embodiment, the microbial composition tailored for those with ABO blood group other than O, comprises or is enriched with DGGE genotype Lacto 14.1%. In a further embodiment, the microbial and/or probiotic composition tailored for those with ABO blood group other than O, comprises or is enriched with DGGE genotype Lacto 14.1% having DNA sequence encoding the 16S rRNA identified as SEQ ID NO:2.

In an embodiment of the invention, the microbial composition is based on bacterial strains corresponding to the DGGE genotypes. The bacterial strains corresponding to each DGGE genotype can be deduced by sequencing the 16S ribosomal DNA (rDNA) from isolated strains with the desired DGGE genotype. As demonstrated in Tables 17 and 18 of Example 17, tailored compositions of *Lactobacillus* and/or *Bifidobacterium* strains for different ABO genotypes of the host can be formulated. As an example, a tailored composition for individuals with blood group O comprises or is enriched with *Lactobacillus* having DGGE genotype 74.2% and DNA sequence encoding the 16S rRNA identified as SEQ ID NO: 4, and/or DGGE genotype 86.6% and DNA sequence encoding the 16S rRNA identified as SEQ ID NO: 7, and/or DGGE genotype 92.3% and DNA sequence encoding the 16S rRNA identified as SEQ ID NO: 8. On the other hand, a tailored composition for individuals with blood group antigens other than O, comprises or is enriched with *Lactobacillus* having DGGE genotype 14.1% and having DNA sequence encoding the 16S rRNA identified as SEQ ID NO: 2.

In one embodiment, the microbial compositions contain only the bacteria specified above as
microbial ingredients, i.e., the microbial compositions consist of the bacteria specified in the preceding paragraphs.

The present invention relates to a method of tailoring a microbial composition for an individual having certain ABO blood
group genotype based on the spectrum of bacteria found in the mucosal tissue of at least one individual with the same ABO blood group genotype. In one embodiment, the present invention relates to a method of tailoring a microbial composition for an individual having A blood group genotype
based on the spectrum of bacteria found in the mucosal tissue of at least one individual with A blood group genotype. In another embodiment, the present invention relates to a method of tailoring a microbial composition for an individual having B blood group genotype based on the spectrum of bacteria found in the mucosal tissue of at least one individual with B blood group genotype. In another embodiment, the present invention relates to a method of tailoring a microbial composition for an individual
having AB blood group genotype based on the spectrum of bacteria found in the mucosal tissue of at least one individual with AB blood group genotype. In a further embodiment, the present invention relates to a method of tailoring a microbial composition for an individual having O blood group genotype based on the spectrum of bacteria found in the mucosal tissue of at least one individual with O blood group genotype.

The microbial composition of the present invention and the supple-ment comprising the composition are particularly suitable and effective, but not limited to, in use for the enhancement of the diversity and numbers of intestinal bacteria, or balancing the microbiota in an individual suffering from immunolog-ical gastrointestinal disorders such as inflammatory bowel disease, celiac disease or irritable bowel syndrome; and/or gastrointestinal disorders or disturbed balance of mucosal microbes as a consequence of or followed by chemotherapy, irradiation or stem cell transplantation, related to the treatment of malignant diseases such as leukemias and/or by subsequent graft-versus-host disease and/or in patients with leukemias.

In one embodiment of the invention, the microbial composition or the supplement comprising the composition is particularly suitable and effective, in use for the enhancement of the diversity and numbers of intestinal bacteria, or balancing the microbiota in an individual suffering from celiac disease. The need for use of targeted microbial modulation in this indication is supported by the findings described in Wacklin P, Pusa E, Kaukinen K, Mäki M, Partanen J, and Mättö J., Composition of the mucosa-associated microbiota in the small intestine of coeliac disease patients and controls. A poster presented in the Rowett-INRA Gut Microbiology-conference, Aberdeen, UK, 22-25.6.2010. It was shown that patients with celiac disease and its different clinical forms have disturbed gut microbiota. Briefly, to evaluate differences between disease symptom groups, intestinal microbiota compositions were assessed from mucosal biopsy samples from coeliac disease patients (n=26; further sub-grouped to gastrointestinal, anemia and dermatitis herpetiformis symptom groups) and from healthy controls (n=25). The samples were analysed by PCR-DGGE with universal bacterial primers. Intestinal microbiota of the coeliac disease patients representing different symptom groups clustered in clearly distinct groups. The finding indicates that the microbiota composition is variable between individuals and in intestinal disorders disease-group related differences in the microbiota composition exist, which should be taken into consideration in applications targeting for balancing or modulating the intestinal microbiota of individuals suffering from immunological gastrointestinal disorders.

In another embodiment of the invention, the microbial composition or the supplement comprising the composition is particularly suitable and effective, in use for the enhancement of the diversity and numbers of intestinal bacteria, or balancing the microbiota in an individual suffering from
gastrointestinal disorders or disturbed balance of mucosal microbes followed by total body irradiation therapy and/or chemotherapy and/or stem cell transplantation and/or by subsequent graft-versus-host disease.

The need for use of targeted microbial modulation in these indications is supported by the findings described in Pusa E, Taskinen M, Lahteen-mäki K, Kaartinen T, Partanen J, Vettenranta K, Mättö J., Do intestinal bacteria or donor derived responsiveness to microbial stimuli play a role post allogeneic HSCT?, a poster presented in the 7^{th} Meeting of the EBMT Paediatric Diseases Working Party, 2-4 June, 2010 Helsinki, Finland. It was demonstrated that the balance of the gut microbiota in these patients was disturbed. Briefly, to evaluate the intestinal microbiota disturbance following chemotherapy or irradiation treatments related to treatments of malignant diseases intestinal microbiota composition of hematopoietic transplantation patients was monitored. Faecal samples were collected from pediatric HSCT patients (before transplantation and 1 wk, 2 wk, 1 mo, 2, mo, 4 mo and 6 months after transplantation) and the corresponding transplant donors. Microbiota profiling was performed by applying standard PCR-DGGE. PCR-DGGE analysis revealed remarkable instability of the intestinal microbiota after transplantation (based on the analysis of samples from three HSCT patients). The similarity of the dominant microbiota was extremely low during the first month after transplantation while up to 94% similarity was detected between the samples obtained 4-6 months from the transplantation. PCR-DGGE specific bacterial group targeted primers revealed absence of several common intestinal bacteria (e.g. bifidobacteria, lactobacilli, C. *leptum* group) in several samples obtained within one month from transplantation. These findings indicate a drastic disturbance of the intestinal microbiota during HSCT and a need for targeted microbiota modulation in these patients.

Further, in one embodiment the present invention relates to a method of tailoring a microbial composition for an individual having certain ABO blood group genotype and having received hematopoietic stem cell graft based on the spectrum of bacteria found in the mucosal tissue of at least one individual with the same ABO blood group genotype. In another embodiment, the present invention relates to a method of tailoring a microbial composition for an individual having certain ABO blood group genotype and celiac disease based on the spectrum of bacteria found in the mucosal tissue of at least one individual with the same ABO blood group genotype.

The microbial composition and the supplement thereof are based on the rationale that those species of bacteria that can be detected in an individual having certain ABO blood group can better or more effectively attach themselves to and/or grow on the mucous having the same ABO blood group antigen than those missing that ABO blood group antigen.

The term "mucosal tissue" here refers to oro-gastrointestinal, gut, skin, oral, respiratory and/or uro-genital tissues or samples derived from these.

The terms "microbial" and "bacterial" here are used as synonyms and refer to any bacterial or other microbial species, strains or their combinations, with health supportive effects, not limited to strains currently accepted as probiotics.

The terms "microbial composition or microbial product" here refers to a probiotic or prebiotic product, including those applied by other routes than the traditional ingested probiotic, e.g. applied directly onto mucosal tissues such as skin or uro-genital tract.

The term "tailoring" refers to determining the ABO blood type of the recipient of the bacterial composition and the typical mucosal bacterial repertoire of the specific ABO blood type by methods known in the art and optionally manufacturing a bacterial composition based on the determined bacterial repertoire. Alternatively, it refers to determining the typical mucosal bacterial repertoire of the specific ABO blood type and the ABO blood type of the recipient by methods known in the art and optionally manufacturing a bacterial composition based on the determined bacterial repertoire. After this tailoring step the typical bacteria or the bacterial composition is administered to the recipient.

The term "personally tailored" refers to targeted modulation based on the ABO blood group genotype of an individual.

The term "probiotic" here refers to any microbial species, strain or their combinations, with health supportive effects, not limited to currently accepted strains or to intestinal effects. The probiotic as defined here may be applied also by other routes than by ingestion, e.g. by applying directly to desired tissue.

The probiotic compositions and supplements so designed may have beneficial effects on the health and/or well-being of a human and may be in the form of, for example, food, capsule or powder. The preparation can be formulated into functional food products or nutritional supplements as well as capsules, emulsions, or powders.

The term "prebiotic" here refers any compound, nutrient, or additional microbe applied as a single additive or as a mixture, together with probiotics or without probiotics, in order to augment a desired probiotic health effect or to stimulate the growth and activity of those bacteria in the digestive system which are assumed to be beneficial to the health of the body. A typical prebiotic ingredient is an oligo/polysaccharide which is non-digestible in the upper parts of the oro-gastrointestinal tract. These oligosaccharides include, but are not limited to, fructo-oligosaccharides or inulin, galacto-oligosaccharides, soy oligosaccharides, resistant starch, and polydextrose. Prebiotics are typically produced by processing from natural sources e.g. from chicory root or milk, alternatively, they may be chemically synthesized. The daily dose needed for a prebiotic effect is typically several grams per day.

In one embodiment of the invention, the microbial composition or a supplement comprising the composition is tailored for individuals belonging to A or AB blood group genotypes. In another embodiment of the invention, the microbial composition or a supplement comprising the composition is tailored for individuals belonging to B or AB blood group genotypes. In a further embodiment of the invention, the microbial composition or a supplement comprising the composition is tailored for individuals belonging to A blood group genotype. In an even further embodiment of the invention, the microbial composition or a supplement comprising the composition is tailored for individuals belonging to B blood group genotype. And finally, in one embodiment of the invention, the microbial composition or a supplement comprising the composition is tailored for individuals belonging to O blood group genotype. The microbial and/or probiotic composition or a supplement comprising the composition can be used to enhance the development of a balanced mucosal e.g. intestinal microbe composition.

The microbial compositions have beneficial effects on the health and/or well-being of a human and may be in the form of, for example, a food product, capsule, tablet or powder. The composition can be formulated into a product of dairy or beverage industry, a functional food product or a nutritional supplement as well as a capsule, emulsion, or powder.

A typical probiotic ingredient is freeze-dried powder containing typically 10¹⁰-10¹² viable probiotic bacterial cells per gram. In addition, it normally
contains freeze drying carriers such as skim milk, short sugars (oligosaccharides such as sucrose or trehalose). Alternatively, the culture preparation can be encapsulated by using e.g. alginate, starch, xanthan as a carrier. A typical probiotic supplement or capsule preparation contains approximately 10⁹-10¹¹ viable probiotic bacterial cells per capsule as a single strain or multi-strain combination.

A typical probiotic food product, which can be among others fermented milk product, fermented milk-based product or juice, contains approximately 10⁹-10¹¹ viable probiotic bacterial cells per daily dose. Probiotics are incorporated in the product as a probiotic ingredient (frozen pellets or freeze dried powder) or they are cultured in the product, such as yogurt, curd and/or sour milk, during fermentation.

The results of the present invention indicated that individuals having different ABO blood group genotypes had different bacterial diversity in their intestines. For example, individuals having B or AB blood group genotypes had different bacterial diversity in their intestines than individuals having A or O blood group genotypes. Thus, among the bacterial strains there were ones, that were more common in the intestine of individuals having B or AB blood group genotypes than individuals having A or O blood group genotypes or strains that were more common in the intestine of individuals having A or AB blood group genotypes than individuals having B or O blood group genotypes or strains that were more common in the intestine of individuals having A, B or AB blood group genotypes than individuals having O blood group genotype.

The invention will be described in more detail by means of the following examples. The examples are not to be construed to limit the claims in any manner whatsoever.

### Examples

### Materials and methods

The materials and methods described herein are common to Examples 1 to 9.

73 healthy adult volunteers (66 females and 9 males) were recruited to the study. Both faecal and blood samples were collected from 71 volunteers (64 females and 9 males). The age of the volunteers ranged from 31 to 61 and was in average 45 years. ABO blood group status was determined from the blood samples using the standard in-house blood grouping protocols of Finnish Red Cross Blood Service.

Faecal samples were frozen within 5 hours from defecation. Bacterial genomic DNA from 1 g of faecal material was extracted using a method described earlier (Apajalahti JH, et al., 1998). This method enables the extraction of intact genomic microbial DNA-strands from faecal samples.

The genomic DNA in bacteria was profiled using %G+C-profiling technique, which allows the identification and fractionation of bacterial clusters in the sample (Apajalahti JH, et al. 1998). The method is based on molecular weight difference between A-T and G-C linkages in DNA double helix. DNA strands consisting of higher concentration of G-C residues, are heavier than AT rich strands, and these can be separated in a salt gradient with ultra speed centrifugation. Analysis of short chain fatty acids (SCFA) and lactic acid was performed essentially as described by Mäkivuokko et al. (Biosci Biotechnol Bi-ochem. 2006; 70: 2056-63) and Fava et al. (Br J Nutr. 2007; 98 :123-33) using gas chromatography to analyse the concentration of SCFAs acetic, propionic, butyric, isobutyric, valeric, isovaleric and 2-methylbutyric acids, as well as lactic acid.

Bifidobacteria genus specific (Mäkivuokko H et al., Nutr Cancer. 2005; 52: 94-104) primers and probes labeled with TaqMan® methodology (Applied Biosystems, Foster City, CA) and quantitative real-time polymerase chain-reaction (qPCR) were used to quantitate the proportion of total bifidobacterial DNA from the entire genomic DNA sample.

For PCR-DGGE analysis DNA from 0.3 g of faecal material was extracted by using the FASTDNA® SPIN KIT FOR SOIL (Qbiogene). PCR-DGGE methods were optimised to detect dominant eubacteria (universal group), *Eubacterium rectale-Clostridium coccoides* (EREC) group, *Bacteroides fragilis* group, *Clostridium leptum* group, *Lactobacillus* group and *Bifidobacterium* group. Partial eubacterial 16S rRNA gene was amplified by PCR with group specific primers (Table 1). The controls are shown in Table 2. Amplified PCR fragments were separated in 8% DGGE gel with denaturing gradient ranging from 45% to 60%. DGGE gels were run at 70 V for 960 mins. DGGE gels were stained with SYBRSafe for 30 mins and documented with Safelmager Bluelight table (Invitrogen) and Alphalmager HP (Kodak) imaging system.

**Table 1**

| Primers used in the DGGE. | | |
|---|---|---|
| **Target group** | **Primer** | |
| universal | U-968-F-GC | Vanhoutte et al. 2004. FEMS Microb Ecol 48, 437-446 |
| universal | U-1401-R | Vanhoutte et al. 2004. FEMS Microb Ecol 48, 437-446 |
| Lactobacillus | Lac1 | Vanhoutte et al. 2004. FEMS Microb Ecol 48, 437-446 |
| Lactobacillus | Lac2GC | Vanhoutte et al. 2004. FEMS Microb Ecol 48, 437-446 |
| EREC | CcocF | Matsuki et al. 2004. Applied and Environmental Microbiology 70, 7220-7228 |
| EREC | CcocR-GC | Matsuki et al. 2004. Applied and Environmental Microbiology 70, 7220-7228 |
| B. fragilis | BfraF | Matsuki et al. 2004. Applied and Environmental Microbiology 70, 7220-7228 |
| B. fragilis | BfraR-GC | Matsuki et al. 2004. Applied and Environmental Microbiology 70, 7220-7228 |
| C. leptum | Clept-F | Matsuki et al. 2004. Applied and Environmental Microbiology 70, 7220-7228 |
| C. leptum | CleptR3-GC | Matsuki et al. 2004. Applied and Environmental Microbiology 70, 7220-7228 |
| Bifidobacterium | Bif164F | Satokari et al. 2001. AEM 67, 504-513 |
| Bifidobacterium | Bif662R-GC | Satokari et al. 2001. AEM 67, 504-513 |

**Table 2**

| The optimised DGGE gel gradients, electrophoresis running conditions for the each bacterial group and the strains used as standards. | | | | |
|---|---|---|---|---|
| Bacterial group | primers* | DGGE gel gradient | Electrophoretic running conditions in Dcode system (Bio-Rad) | Strains in standard |
| Universal | U968F-GC, U1401R | 38-60% | 70 V, 960 mins | *A*. *cacae* DSM 14662 |
| | | | | C. *perfringens* DSM 756 |
| | | | | *E. ramulus* DSM 15687 |
| | | | | *F. prausnitzii* DSM 17677 |
| | | | | *E. coli* DSM 30083 |
| | | | | *L. rhamnosus* DSM 96666 |
| | | | | *P. melaninogenica* DSM 7089 |
| Bifidobacterium | Bif164F, Bif662R-GC | 45-60% | 70 V, 960 mins | *B. adolescentis* DSM 981074 |
| | | | | *B. angulatum* DSM 20098 |
| | | | | *B. longum* DSM 96664 |
| | | | | *B. catenulatum* DSM 16992 |
| | | | | *B. lactis* DSM 97847 |
| Lactobacillus | Lac1, Lac2-GC | 38-55% | 70 V, 960 mins | *L. plantarum* E-79098 |
| | | | | *L. cellubiosis* E-98167 |
| | | | | *L. reuterii* E-92142 |
| | | | | *L. paracasei* E-93490 |
| B.fragilis | BfraF, BfraR-GC | 30-45% | 70 V, 960 mins. | *B. caccae* DSM 19024 |
| | | | | *B. uniformis* DSM 6597 |
| | | | | *B. eggerthii* DSM 20697 |
| EREC | CcocF; CcocR-GC | 40-58% | 70V, 960 mins | *L. multipara* DSM 3073 |
| | | | | *A*. *cacae* DSM 14662 |
| | | | | *D. longicatena* DSM 13814 |
| | | | | *R. productus* DSM 2950 |
| C.leptum | CleptF, CleptR3-GC | 30-53% | 70V, 960 mins | *F. prausnitzii* DSM 17677 |
| | | | | *C. methylpentosum* DSM 5476 |
| | | | | *R. albus* DSM 20455 |
| | | | | *C. leptum* DSM 753 |
| | | | | *E. siraeum* DSM 15702 |
| | | | | *C. viridae* DSM 6836 |

Digitalised DGGE gel images were imported to the Bionumerics-program version 5.0 (Applied Maths) for normalisation and band detection. The bands were normalised in relation to a marker sample specific for the said bacterial groups. Band search and bandmatching were performed as implemented in the Bionumerics. Bands and bandmatching were manually checked and corrected. Principal component analysis was calculated in the Bionumerics. Anova and Kruskal-Wallis test were computed with statistical programming language R, version 2.8.1. The Fisher's exact test was calculated using the StatsDirect program, version 2.5.6 (StatsDirect Ltd.)

The bands were excised from DGGE gels. DNA fragments from bands were eluted by incubating the gel slices in 50 µl of sterile H₂O at +4 °C overnight. The correct positions and purity of the bands were checked for each excised bands by amplifying DNA in bands and re-running the amplified fragments along with the original samples in DGGE. Bands which produced single bands only and were in the correct position in the gels were sequenced. The sequences were trimmed, and manually checked and aligned by ClustalW. The closest relatives of the sequences were searched using NCBI Blast v2.2.21 and nr database (www.ncbi.nlm.nih.gov/Blast, searched on 1.10.2009). Distance matrix of the aligned sequences was used to compare the similarity of the sequences.

Blood group adhesion of lactobacilli and bifidobacteria was evaluated with a liquid/solid phase assay. The studied bacterial strains were allowed to adhere on ABO glycan structures with a biotin tail in a liquid phase. After the initial adhesion, the bacterial suspensions were placed in 96-well with streptavidin coated wells. The biotin molecules of the glycan structures have a high affinity towards the streptavidin, thus initialising the second adhesion step, which attaches the glycan-microbe-complex to the wells. The microbes were labelled with DNA-specific dye (e.g. Syto 9, Invitrogen) and the intensity of the dye was monitored after the second adhesion. Results were compared to blood group A glycan adherence of a previously reported blood group A specific strain, *Lactobacillus crispatus* LMG18199 and the strains with same or higher fluorescence measurements on any ABO glycan, were selected as potentially adhering strains.

The findings presented in Examples 1 to 9 were re-tested by collecting additional sample material; the number of individuals in examples 10 to 17 was 64 (21 = blood group A; 13 = B; 11 = AB; 19 = O). The analyses of the additional samples were performed as described in Examples 1 to 9, if not specified.

The samples that did not amplify in the PCR step of specific DGGE analysis in spite of proper PCR amplification in the Universal DGGE (6 sam-ples in Bifidobacteria DGGE, 3 in BFRA and 1 in Lactobacillus) were handled as zeros in the data analysis.

Redundancy analysis (RDA) is a multivariate ordination analysis, basically a Principal Component Analysis in which the axes are restricted to be linear combinations of explanatory variables, where results indicate linear species-environmental relationships (in the present application bacteria-blood group relationships).

### Example 1

ABO blood group status was determined from the blood samples using the standard in-house blood grouping protocols of Finnish Red Cross Blood Service. The distribution of blood groups was: 25 A, 23 O, 11 B and 14 AB, a total of 73 samples.

### Example 2

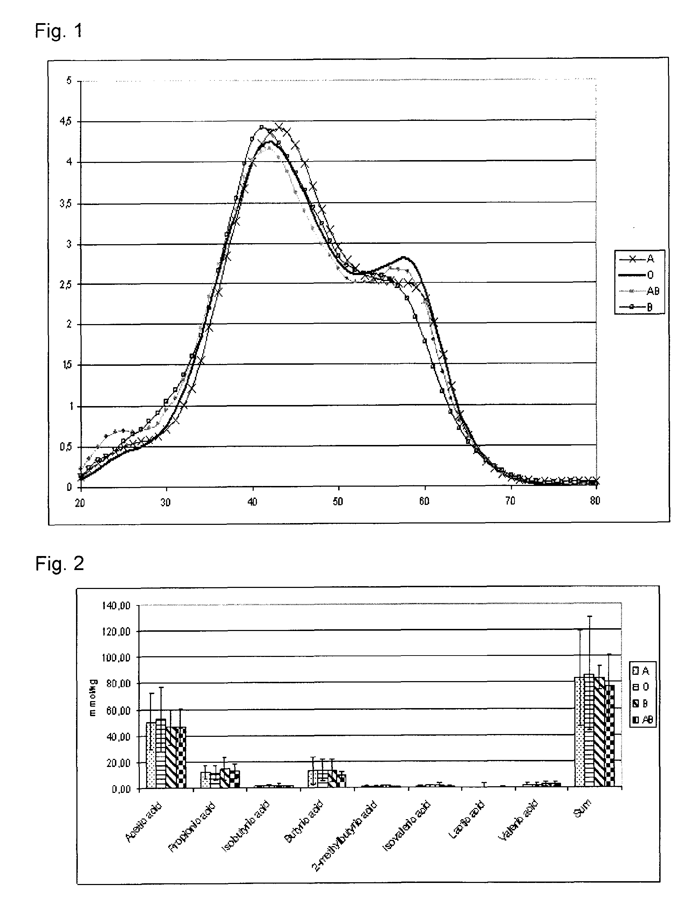

In %G+C-profiling analysis of genomic bacterial DNA in the samples (Figure 1), several statistically significant differences (p < 0.05) between ABO blood groups were found (Table 3), when averaged single sample profiles were divided into increments of 5%-units and the groups were compared pair-wise. The distribution of blood groups were 14 A, 17 O, 9 B and 8 AB, a total of 48 samples could be analysed.

**Table 3**

| Statistical significances between 5 %G+C-fractionated grouped and samples averaged by ABO blood group status. Statistical significances between indicated groups were tested by using Student's t-test. | | | | | | |
|---|---|---|---|---|---|---|
| 5% increment | A vs. O | A vs AB | A vs B | OvsB | O vs AB | AB vs B |
| 20-24 | 0.0003 | 0.0020 | 0.0043 | 0.0013 | 0.0006 | 0.0026 |
| 25-29 | 0.0179 | 0.0001 | 0.0043 | 0.0003 | 0.0003 | 0.3791 |
| 30-34 | 0.0075 | 0.0003 | 0.0000 | 0.0001 | 0.0117 | 0.2795 |
| 35-39 | 0.0058 | 0.0036 | 0.0000 | 0.0039 | 0.0454 | 0.3542 |
| 40-44 | 0.0455 | 0.0340 | 0.4896 | 0.0234 | 0.0218 | 0.0003 |
| 45-49 | 0.0002 | 0.0004 | 0.0022 | 0.0011 | 0.0042 | 0.0000 |
| 50-54 | 0.1471 | 0.0085 | 0.2307 | 0.0001 | 0.0856 | 0.0003 |
| 55-59 | 0.0004 | 0.0055 | 0.1376 | 0.0032 | 0.0110 | 0.0204 |
| 60-64 | 0.0210 | 0.0091 | 0.0022 | 0.0010 | 0.0022 | 0.0076 |
| 65-69 | 0.0120 | 0.0769 | 0.4867 | 0.1672 | 0.1661 | 0.1746 |
| 70-74 | 0.0117 | 0.2943 | 0.2289 | 0.0080 | 0.0079 | 0.0235 |

### Example 3

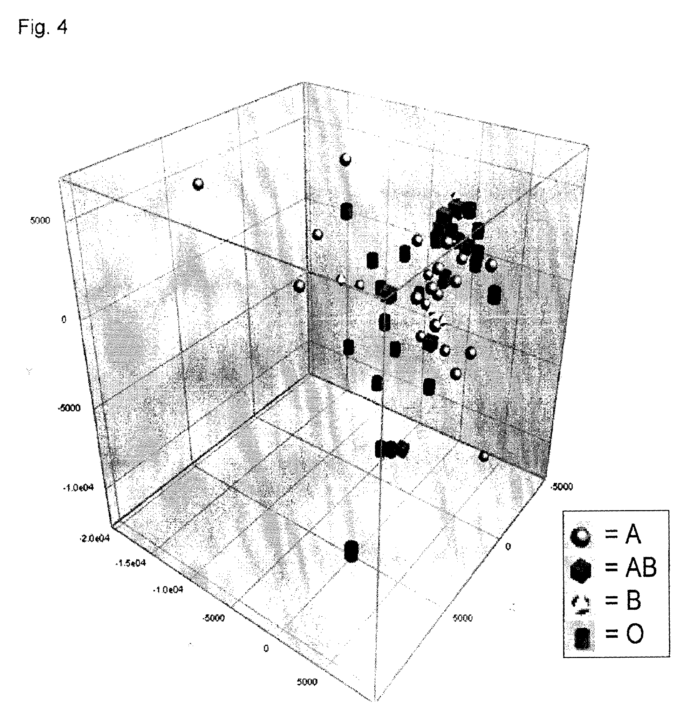

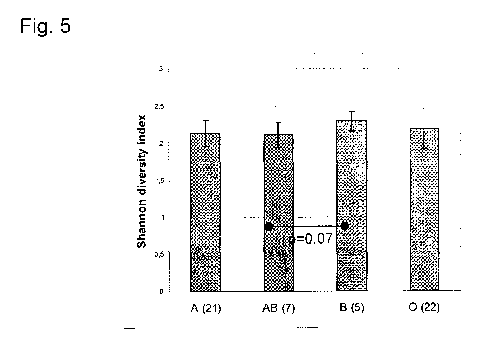

In the universal DGGE analysis of dominant intestinal bacteria, two genotypes, band positions 18.40% and 31.20% showed different frequencies between the samples obtained from different blood groups (Table 4). Principal component analysis (PCA analysis) showed no clear blood-group associated clustering of the samples (Figure 4). Samples from blood group B individuals tended to have a higher diversity, especially as compared with those from blood group AB (p = 0.07) (Figure 5).

**Table 4**

| Statistically significant differences on band intensities between ABO blood groups as determined by universal-DGGE (N=52 samples with blood groups A = 21 samples, B = 5, AB = 7 and O = 22). Statistical tests, ANOVA (ANO) and Kruskal-Wallis (KW) were based on band intensity matrix. | | | | |
|---|---|---|---|---|
| **Genotype** | **Test** | **p-value** | **#of hits** | **Distribution according to ABO blood group** |
| 18.40% | ANO/KW | 0.03/0.03 | 4 | B (33%); AB (13%); 0 (5%); A (0%) |
| 31.20% | KW | 0.03 | 20 | AB (63%); A (48%); 0 (18%); B (0%) |

### Example 4

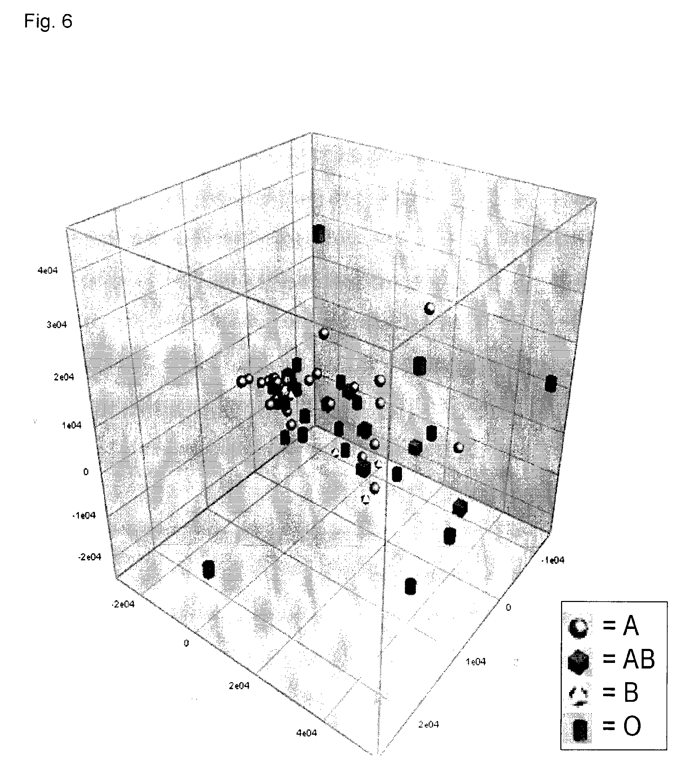

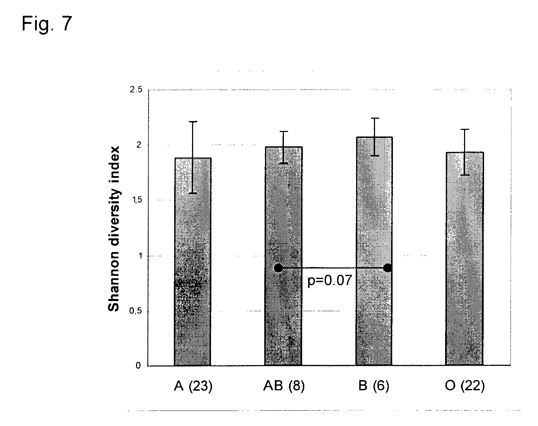

A genotype belonging to *Eubacterium rectale-Clostridium coccoides-* group (EREC) and corresponding band position 61.10% in the EREC-DGGE gels was more frequently detected in the samples with AB blood group (63%) than in other blood groups. The lowest frequency was in the blood group A individuals (13%). The results are shown in Table 5. PCA analysis did not show clear blood-group associated clustering of the samples (Figure 6). Samples from blood group B individuals tended to have a higher diversity, especially as compared with those from blood group AB (p = 0.07) (Figure 7).

**Table 5**

| Statistically significant differences on band intensities between samples obtained from different ABO blood groups as determined by EREC-DGGE. Statistical testing by using Kruskal-Wallis (KW) was based on the band intensity matrix. | | | | |
|---|---|---|---|---|
| **Genotype** | **Test** | **p-value** | **#of hits** | **Distribution according to ABO blood group** |
| 61.10% | KW | 0.03 | 17 | AB (63%); B(33%); O(32%); A(13%) |

### Example 5

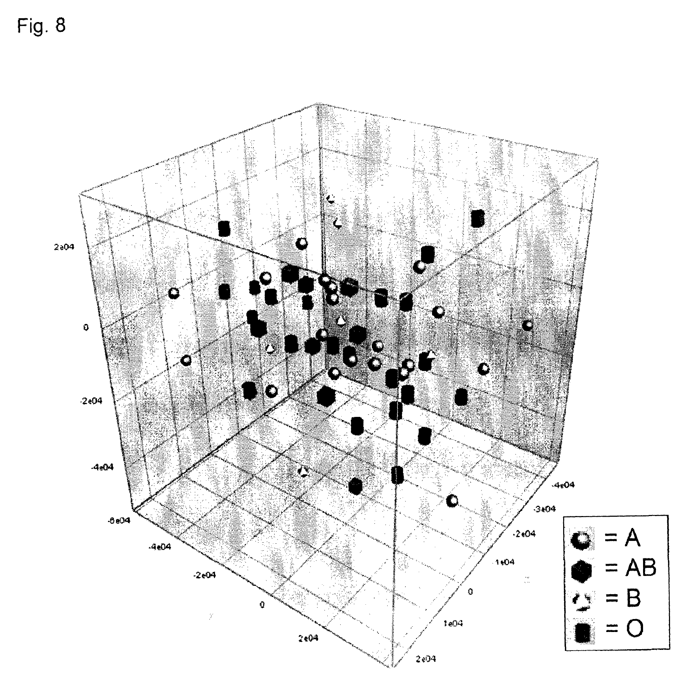

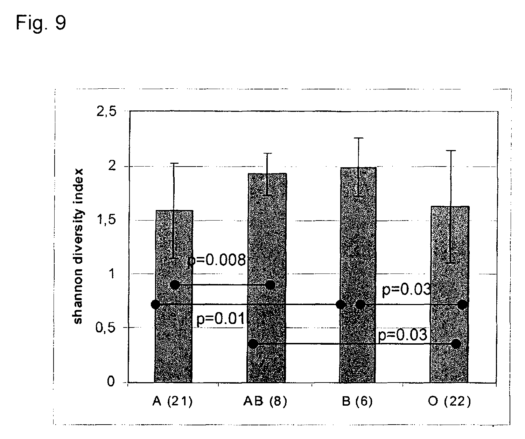

Two genotypes of *Bacteroides fragilis* group, 25.90% and 41.10%, had statistically significant differences in detection frequency between ABO blood groups (Table 6). PCA analysis did not show clear blood-group associated clustering of the samples (Figure 8). Diversity of *B*. *fragilis* group PCR-DGGE profiles was significantly higher in samples with blood groups B and AB than in those with blood groups A and O (Figure 9).

**Table 6**

| Statistically significant differences on band intensities between samples obtained from different ABO blood groups as determined by *B. fragilis* group DGGE. Statistical tests, ANOVA (ANO) and Kruskal-Wallis (KW) were based on the band intensity matrix. | | | | |
|---|---|---|---|---|
| **Genotypes** | **test** | **p-value** | **#of hits** | **Distribution according to ABO blood group** |
| 25.90% | ANO/KW | 0.004/0.007 | 9 | B (50%); AB (38%); 0 (14%); A (0%) |
| 41.10% | KW | 0.01 | 7 | B (50%); AB (25%); 0 (9%); A (0%) |

### Example 6

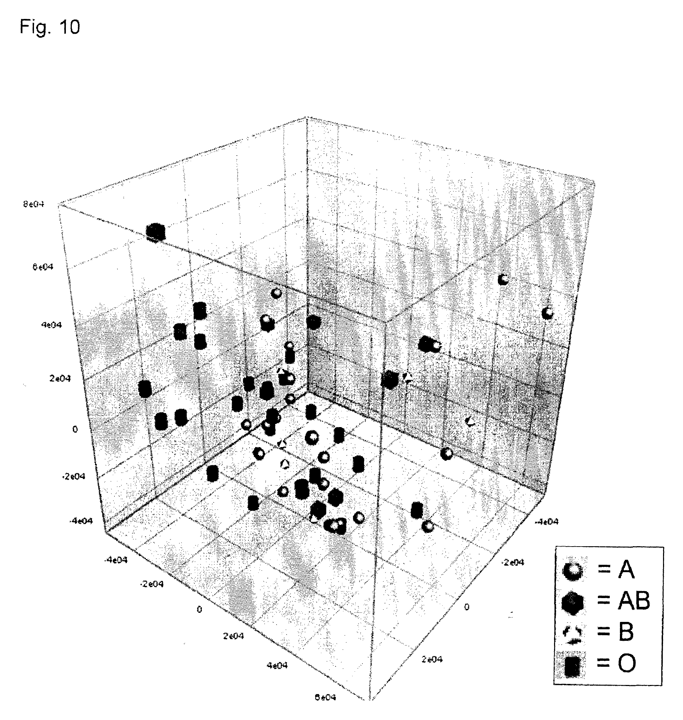

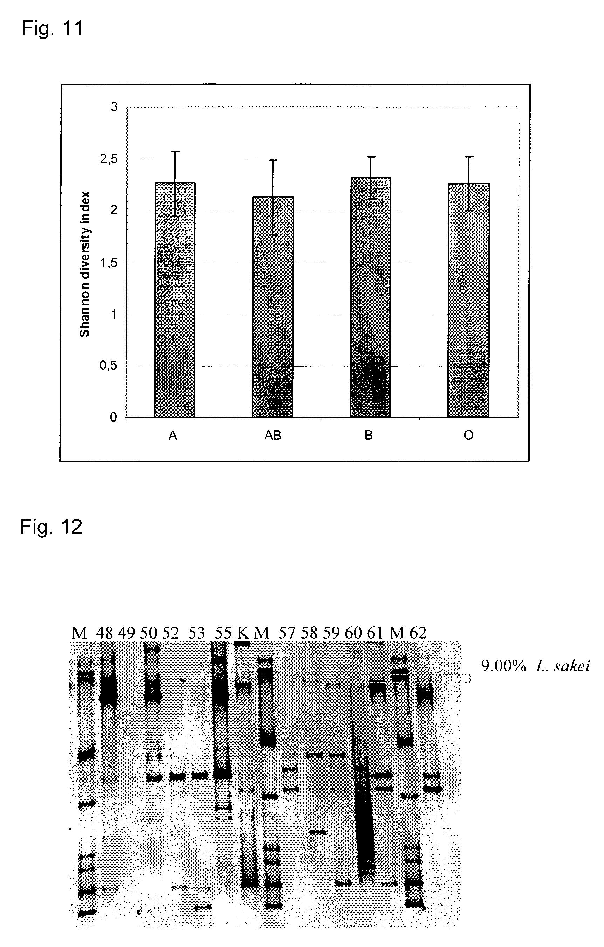

In one genotype of *Clostridium leptum* group statistically significant difference in detection frequency between ABO blood groups was observed. The genotype band position 84.10% was detected in 36% of samples obtained from blood group O individuals, while the corresponding figures for A, AB and B blood groups were 7%, 0% and 0%, respectively (Table 7). PCA analysis showed visual clustering of the samples from A and O individuals to opposite directions in the PCA plot (Figure 10). No differences in the diversity of the C. *leptum* group PCR-DGGE profiles were observed between the blood groups (Figure 11).

**Table 7**

| Statistically significant differences on band intensities between ABO blood group samples as determined by C. *leptum* DGGE. Kruskal-Wallis (KW) was based on band intensity matrix. | | | | |
|---|---|---|---|---|
| **Genotype** | **test** | **p-value** | **#of hits** | **Distribution according to ABO blood group** |
| 84.10% | KW | 0.03 | 10 | O(36%); A (9%); AB(0%); B(0%) |

### Example 7

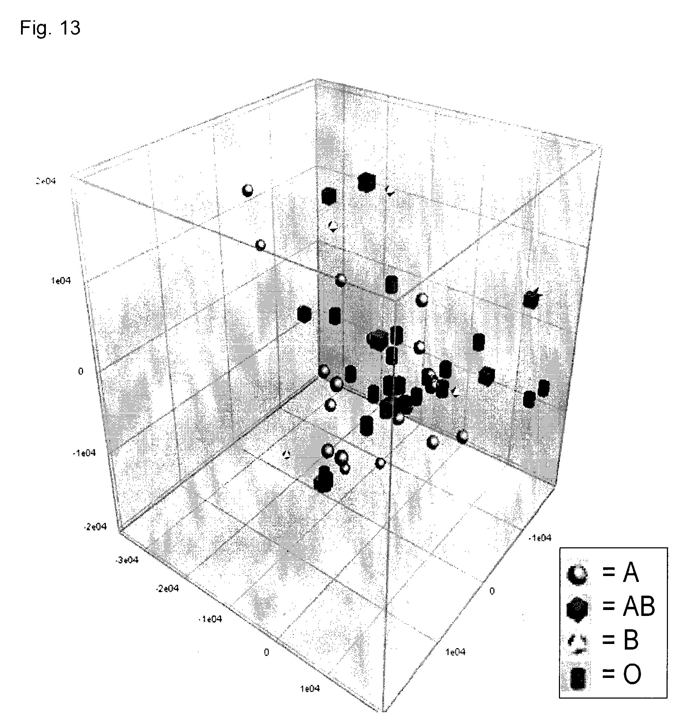

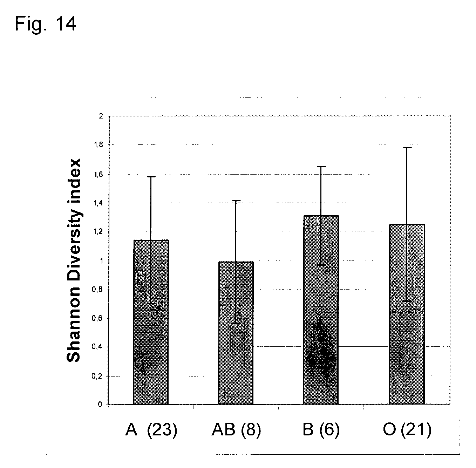

In one genotype of *Lactobacillus* group, 9.00% statistically significant difference in detection frequency between ABO blood groups was observed (Table 8). This genotype was shown to represent *L. sakei* species by partial 16S rDNA sequencing (Figure 12). PCA analysis did not show clear blood-group associated clustering of the samples (Figure 13) and no difference in the diversity in the Lactobacillus group specific PCR-DGGE profiles was observed between the blood groups (Figure 14).

**Table 8**

| Statistically significant differences on band intensities between ABO blood group samples as determined by *Lactobacillus* group-specific DGGE. Statistical test, Kruskal-Wallis (KW) was based on band intensity matrix. F = Fisher's exact test for distribution was based on presence/absence matrix of bands. | | | | |
|---|---|---|---|---|
| **Genotype** | **test** | **p-value** | **#of hits** | **Distribution according to ABO blood group** |
| 9.00% | KW/F | 0.02/0.02 | 13 | B (67%); AB (38%); A (13%); O (14%) |

### Example 8

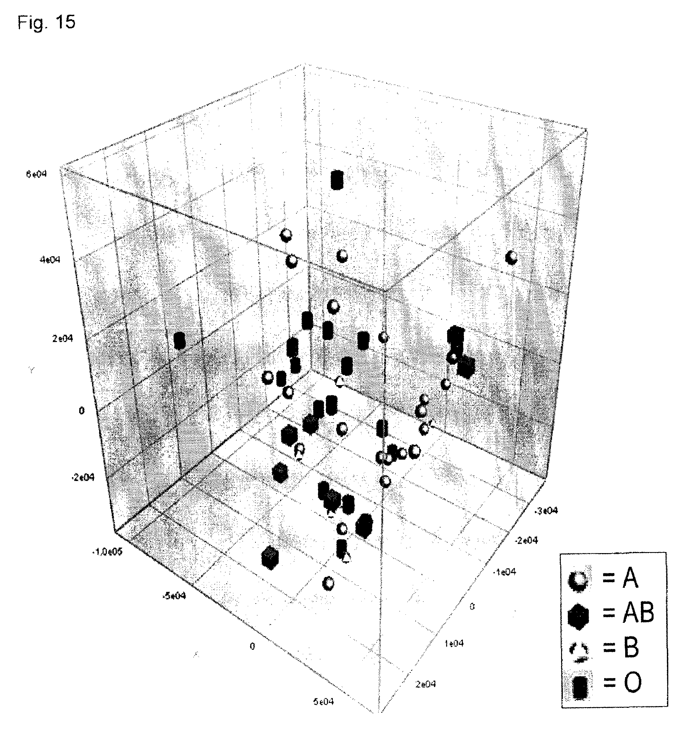

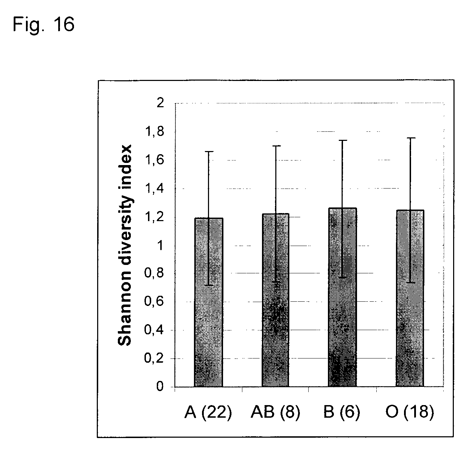

ABO blood group specific Bifidobacteria genotypes were not detected in the *Bifidobacterium* group specific PCR-DGGE (data not shown), further the *Bifidobacterium* group specific PCR-PGGE profiles did not show blood group associated clustering in the PCA analysis (Figure 15) or differences in the diversity of the PCR-DGGE profiles (Figure 16).

### Example 9

Occurrence of several of the DGGE-derived band position genotypes described in the Examples above were significantly associated with the presence of a specific ABO blood group antigen (Table 9). Genotypes universal-DGGE (UNIV) 18.40%, Lactobacillus-DGGE (LACTO) 9.00%, Bacteroides fragilis-DGGE (Bfra) 25.90% and Bfra 41.10% were significantly associated with the presence of blood group B antigen, i.e. samples with ABO types B and AB. Genotype UNIV 31.20%, was statistically significantly associated with the presence of the A antigen, i.e. samples with A or AB *versus* those with B or O status. Genotype C. *leptum* 84.10% was associated with the samples with O antigen (i.e. O *versus* A + B + AB).

**Table 9**

| Association of bacterial genotypes with A, B and O blood group antigens. Only genotypes that showed statistically significant differences in Examples 5 to 10 were tested. Statistically significant P-values as tested by Fisher's exact test are shown in brackets. | | | |
|---|---|---|---|
| **DGGE type, genotype, number of genotype detected** | **B+ AB vs. O + A** | **A + AB vs. O + B** | **O vs. A+AB+B** |
| UNIV, 18.40%, n=4 | 21 % vs. 2% (0.04) | 3% vs. 11% | 5% vs. 8% |
| UNIV, 31.20%, n=20 | 36% vs. 33% | 52% vs. 14% (0.003) | 18% vs. 43% (0.05) |
| Lacto, 9.00%, n=13 | 50% vs. 13% (0.01) | 19% vs. 25% | 14% vs. 30% |
| EREC, 61.10%, n=17 | 50% vs. 22% | 26% vs. 32% | 32% vs. 30% |
| *C. leptum*, 84.10%, n=10 | 0% vs. 22% (0.05) | 6% vs. 29% (0.03) | 36% vs. 6% (0.004) |
| Bfra, 25.90%, n=9 | 43% vs. 7% (0.005) | 10% vs. 21% | 14% vs. 18% |
| Bfra, 41.10% n= 7 | 36% vs. 4% (0.008) | 6% vs. 18% | 9% vs. 15% |

### Example 10

### DGGE with universal primers

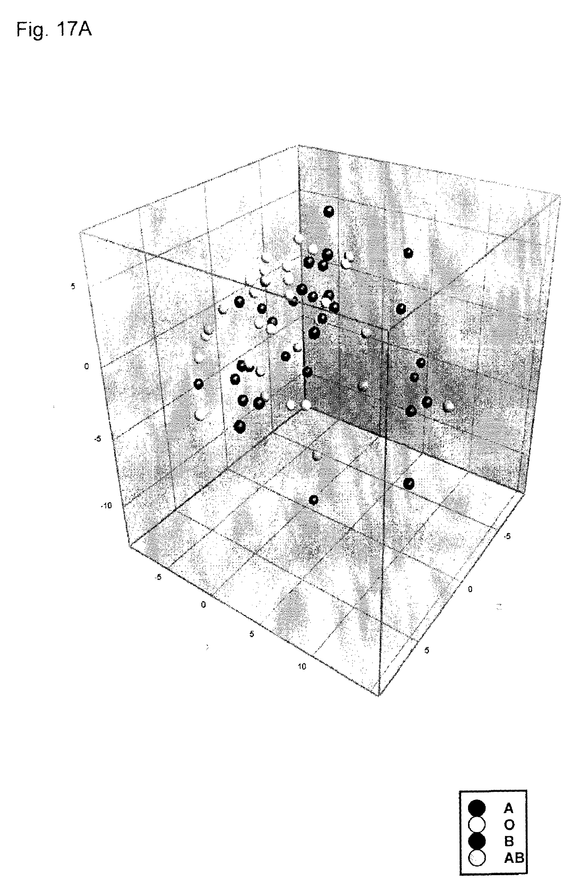

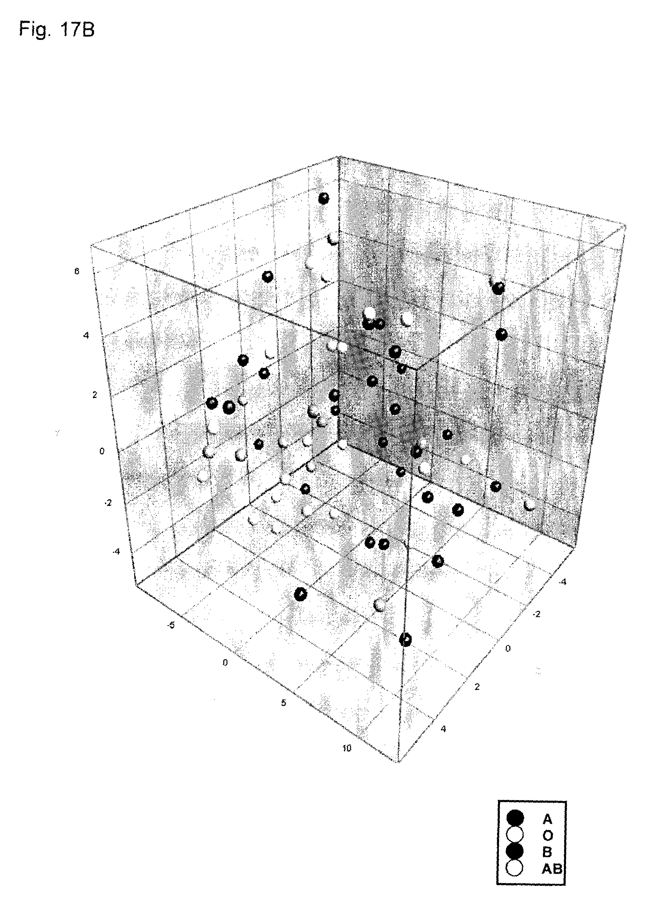

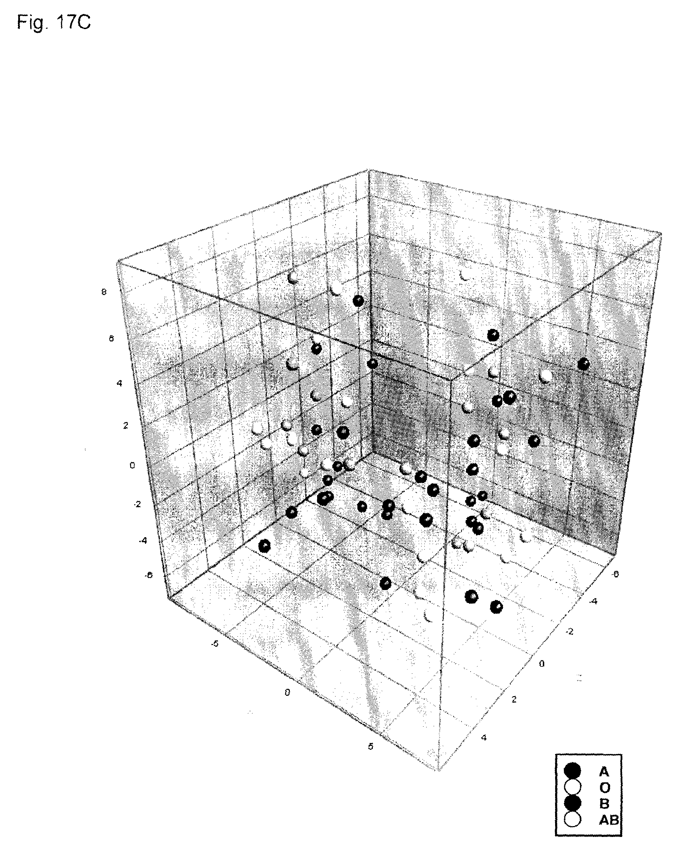

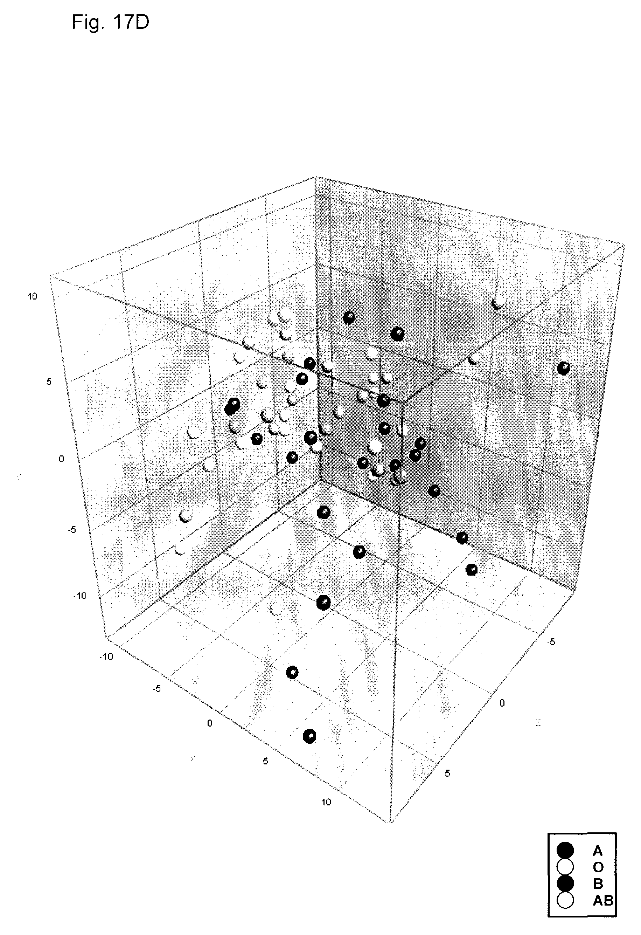

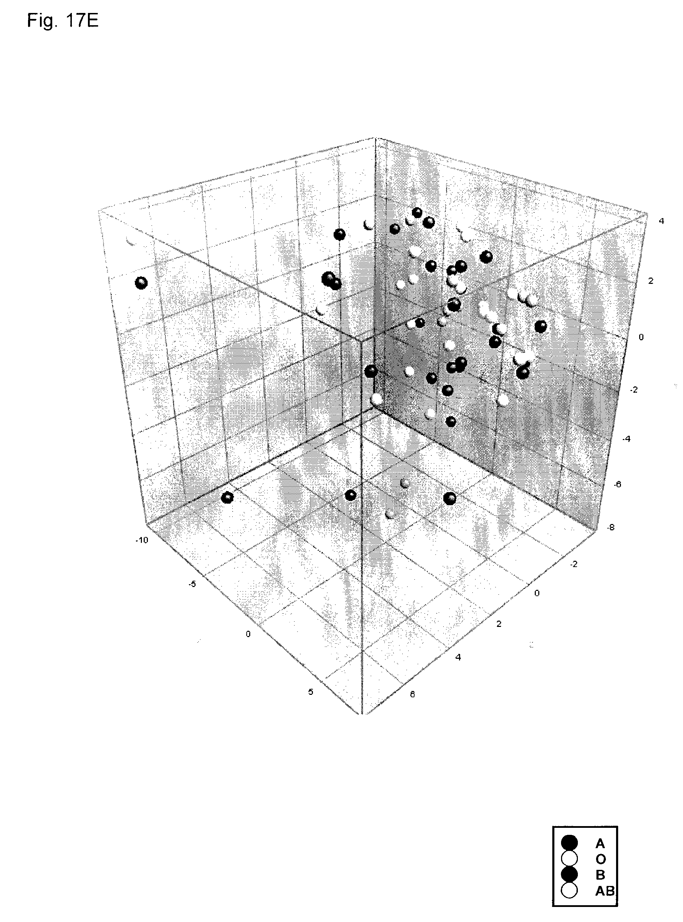

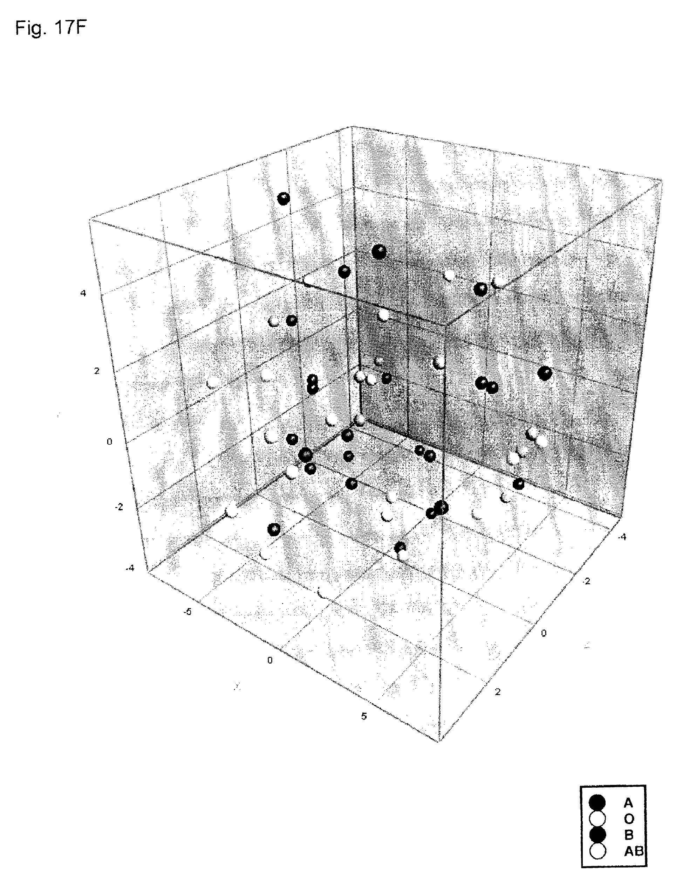

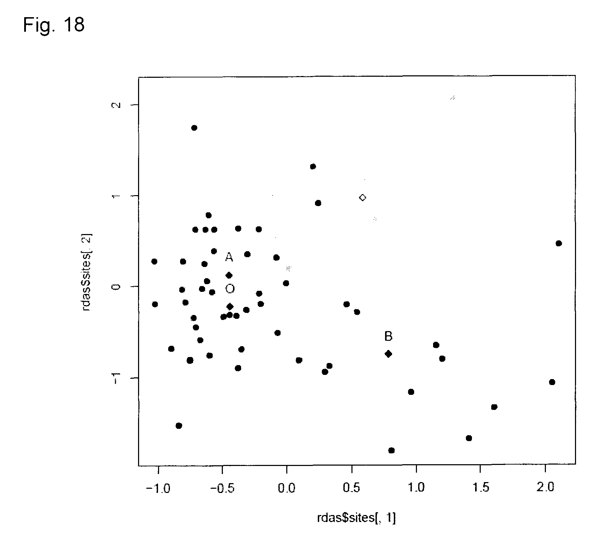

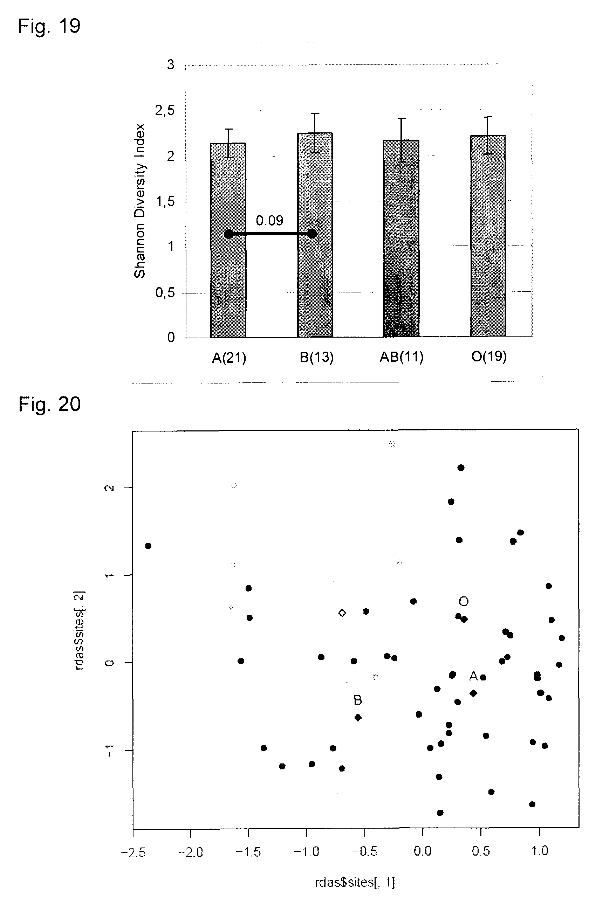

In the universal DGGE analysis of dominant intestinal bacteria, six genotypes, band positions 18.0%, 32.2%, 33.8%, 42.4%, 60.2% and 61.1% showed different frequencies between the samples obtained from different blood groups (Table 10). Principal component analysis (PCA) clustering of the samples is presented in Figure 17. RDA analysis showed distinct ABO blood group specific clustering (p= 0.015) indicating ABO blood group related differences in the dominant microbiota composition (Figure 18). Samples from blood group B individuals had a higher diversity, especially as compared with those from blood group A (p = 0.09) (Figure 19).

**Table 10**

| Statistically significant differences in the band presence between ABO blood groups as determined by universal DGGE analysis (N=64 samples with blood groups A = 21 samples, B = 13, AB = 11 and O = 19). Statistical testing, Fisher's exact test, was based on band presence absence matrix. | | | |
|---|---|---|---|
| **Genotype (band position)** | **p-value** | **number of hits** | **Distribution according to ABO blood group** |
| 18.0% | 0.0004 | | B (46%); AB (18%); 0 (5%); A (0%) |
| 32.2% | 0.004 | | AB (36%); B (23%); O (5%); A (0%) |
| 33.8% | 0.04 | 6 | O (100%); A (90%); B (77%); AB (73%) |
| 42.4% | 0.02 | | A (10%); B (31%); O (0%); AB (27%) |
| 60.2% | 0.046 | 7 | A (19%); B (54%); O (26%); AB (9%) |
| 61.1% | 0.004 | | A (0%); B (8%); O (0%); AB (27%) |

### Example 11

### DGGE with EREC primers

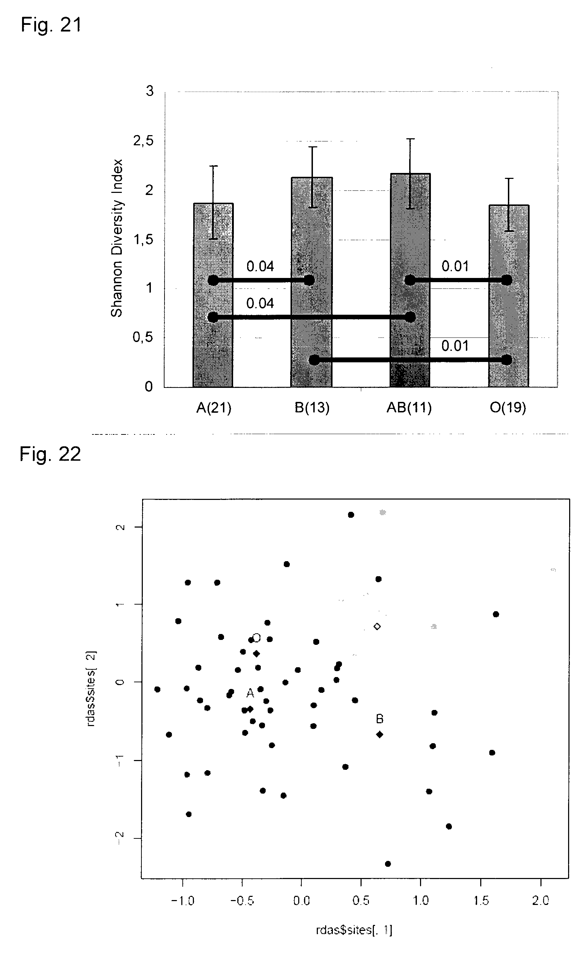

ABO blood group related differences in the detection frequency was observed in four genotypes belonging to *Eubacterium rectale-Clostridium coccoides-* group (EREC). The genotypes corresponded to band positions 4.8%, 35.3%, 46.9% and 77.8% in the EREC-DGGE gels (Table 11). RDA analysis showed clear ABO blood group specific clustering (p= 0.032) indicating ABO blood group related differences in the EREC group composition (Figure 20). Samples from individuals with B-antigen (B and AB blood groups, respectively) had significantly higher diversity compared to both A and O groups (p<0.04) (Figure 21).

**Table 11**

| Statistically significant differences in the band presence between samples obtained from different ABO blood groups as determined by EREC-DGGE. (N=64 samples with blood groups A = 21 samples, B = 13, AB = 11 and O = 19). Statistical tests, Fisher's exact were based on band presence absence. | | | |
|---|---|---|---|
| **Genotype (band position)** | **p-value** | **number of hits** | **Distribution according to ABO blood group** |
| 4.8% | 0.005 | 13 | AB (36%); B(8%); O(5%); A(33%) |
| 35.3% | 0.046 | 8 | A (5%); B (15%); AB (36%); O (5%) |
| 46.9% | 0.006 | 19 | A (24%); B (54%); AB (45%); O (11%) |
| 77.8% | 0.045 | 4 | A (5%); B (23%); AB (0%); O (0%) |

### Example 12

### B. fragilis DGGE

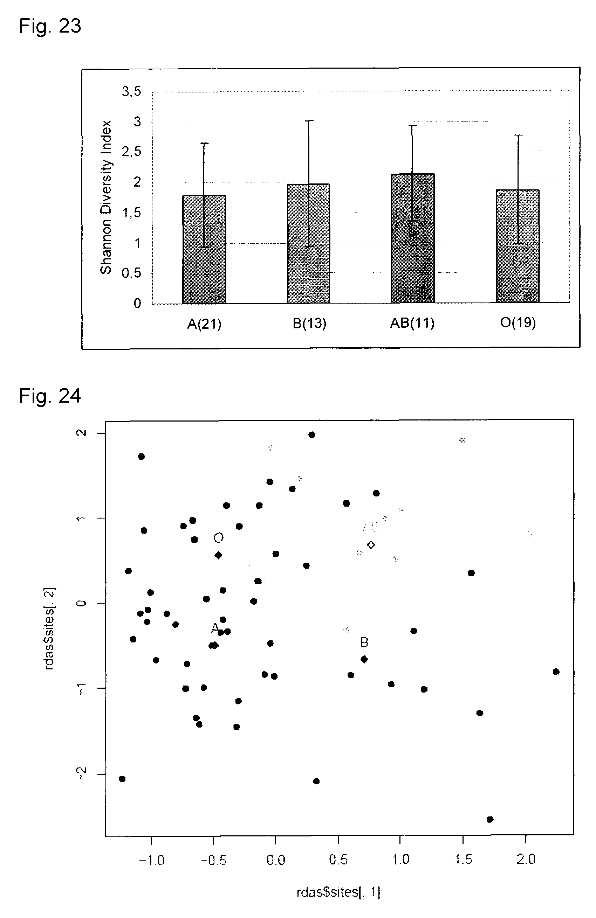

Five genotypes of *Bacteroides fragilis* (BFRA) group, 5.0%, 8.9%, 15.6%, 21.5% and 26.1%, had statistically significant differences in detection frequency between ABO blood groups (Table 12). RDA analysis did not show clear ABO blood-group associated clustering of the samples (Figure 22). No differences in diversity in *B. fragilis* group PCR-DGGE profiles were detected between blood groups (Figure 23).

**Table 12**

| Statistically significant differences in the band presence between samples obtained from different ABO blood groups as determined by *B. fragilis* group DGGE (N=64 samples with blood groups A = 21 samples, B = 13, AB = 11 and O = 19). Statistical testing, Fisher's exact test, was based on band presence/absence. | | | |
|---|---|---|---|
| **Genotype (band position)** | **p-value** | **number of hits** | **Distribution according to ABO blood group** |
| 5.0% | 0.01 | | A (0%); B (23%); O (0%); AB (18%) |
| 8.9% | 0.04 | 9 | A (95%); B (69%); O (79%); AB (55%) |
| 15.6% | 0.047 | | A (5%); B (23%); O (0%); AB (0%) |
| 21.5% | 0.04 | | A (5%); B (38%); O (11%); AB (9%) |
| 26.1% | 0.02 | | A (0%); B (8%); O (16%); AB (36%) |

### Example 13

### C. leptum DGGE

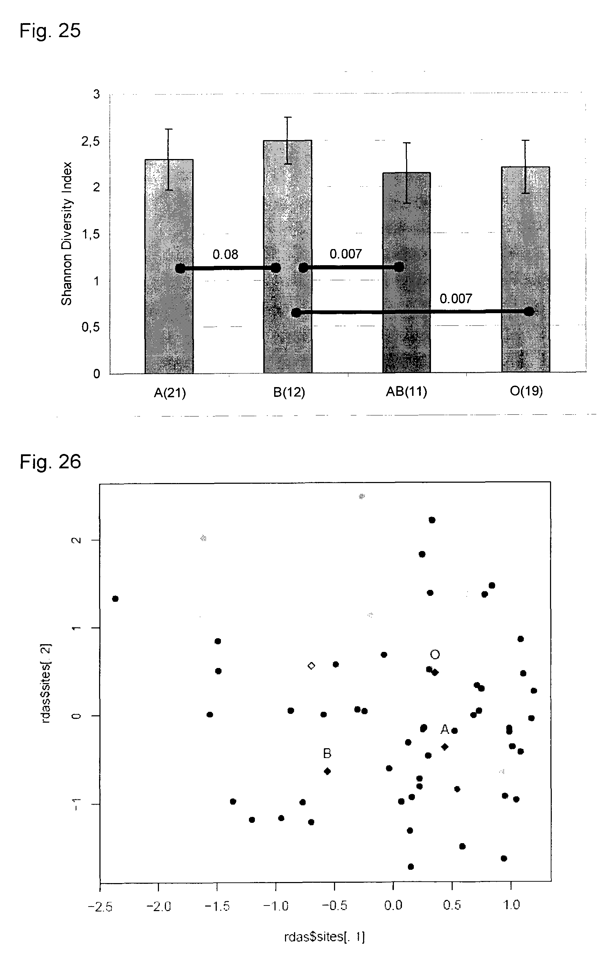

In five genotypes of *Clostridium leptum* group statistically significant difference in detection frequency between ABO blood groups was observed. These genotypes corresponded to band positions 11.9%, 15.4%, 16.0%, 20.5% and 67.9% (Table 13). RDA analysis did not show clear ABO blood group associated clustering of the samples (Figure 24). Samples from individuals with blood group B had a higher diversity in the C. *leptum* group PCR-DGGE profiles compared to the other blood groups, especially compared to AB- and O-groups (p<0.007, respectively) (Figure 25).

**Table 13**

| Statistically significant differences in the band presence between ABO blood group samples as determined by C. *leptum* DGGE. (N=64 samples with blood groups A = 21 samples, B = 13, AB = 11 and O = 19). Statistical testing, Fisher's exact test, was based on band presence/absence. | | | |
|---|---|---|---|
| **Genotype (band position)** | **p-value** | **number of hits** | **Distribution according to ABO blood group** |
| 11.9% | 0.014 | 31 | A (62%); B (31%); O (63%); AB (18%) |
| 15.4% | 0.013 | 8 | A (10%); B (38%); O (5%); AB (0%) |
| 16.0% | <0.0001 | 6 | A (0%); B (8%); O (0%); AB (45%) |
| 20.5% | 0.047 | 9 | A (10%); B (31%); O (5%); AB (18%) |
| 67.9% | 0.04 | 12 | A (14%); B (46%); O (11%); AB (9%) |

### Example 14

### Lactobacillus DGGE

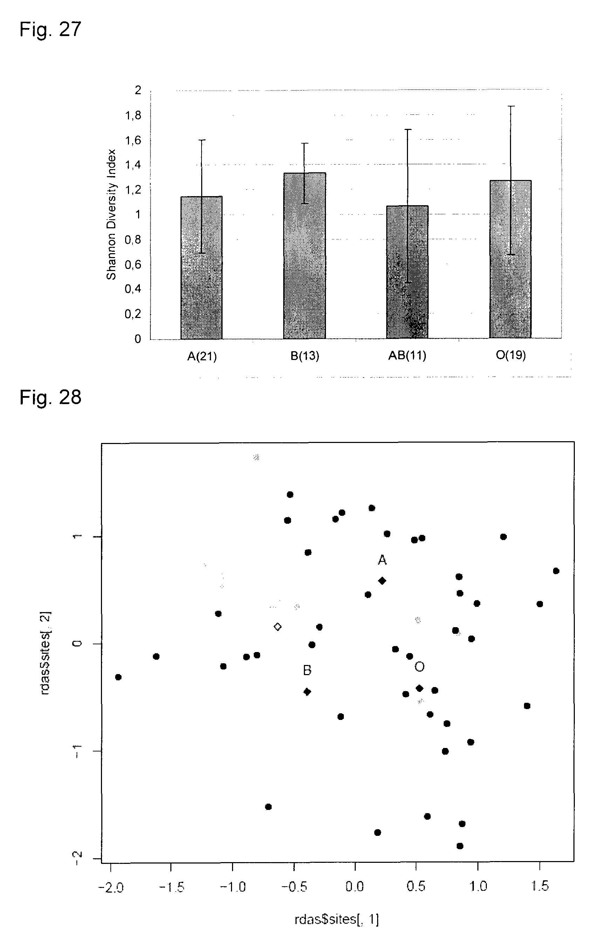

In one genotype of *Lactobacillus* group, 92.3% statistically significant difference in detection frequency between ABO blood groups was observed, and in three additional genotypes, 83.1%, 84.7% and 86.6% a trend-like difference was observed (Table 14). RDA analysis showed clear ABO blood group specific clustering (p= 0.036) indicating differences in the *Lactobacillus* composition (Figure 26). No difference in the diversity in the *Lactobacillus* group specific PCR-DGGE profiles was observed between the blood groups (Figure 27).

**Table 14**

| Statistically significant differences on band presence between ABO blood group samples as determined by *Lactobacillus* group-specific DGGE. (N=64 samples with blood groups A = 21 samples, B = 13, AB = 11 and O = 19). Statistical testing, Fisher's exact test was based on band presence/absence. | | | |
|---|---|---|---|
| **Genotype (band position)** | **p-value** | **number of hits** | **Distribution according to ABO blood group** |
| 83.1% | 0.06 | 4 | A (0%); B (15%); O (0%); AB (0%) |
| 84.7% | 0.06 | 40 | A (48%); B (54%); O (74%); AB (82%) |
| 86.6% | 0.07 | 3 | A (0%); B (0%); O (16%); AB (0%) |
| 92.3% | 0.03 | 8 | A (5%); B (0%); O (32%); AB (9%) |

### Example 15

### Bifidobacterium DGGE

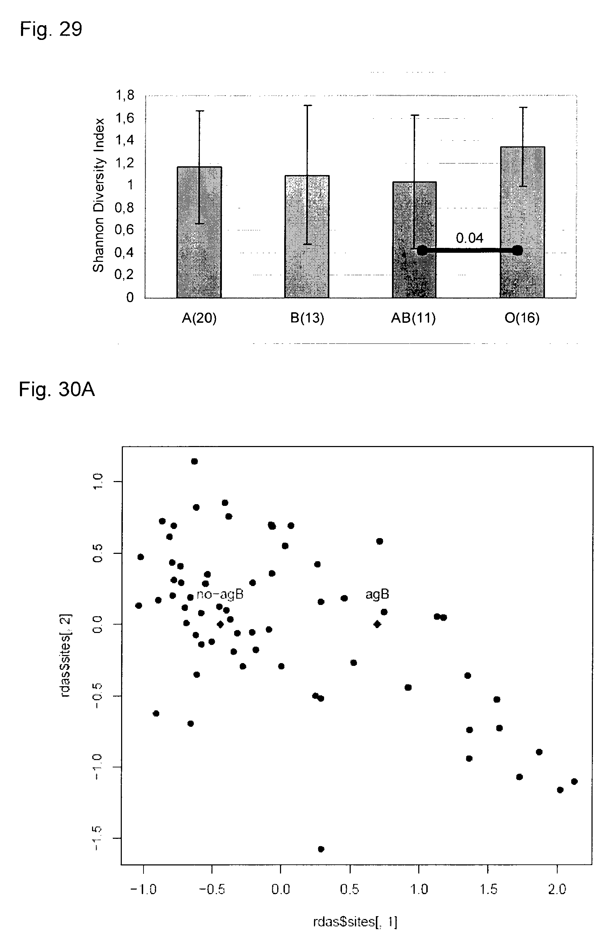

One ABO blood group specific Bifidobacteria genotype corresponding to the band position 26.6% was detected in the *Bifidobacterium* group specific PCR-DGGE (Table 15). In addition, two genotypes corresponding to band positions 24.9% and 53.5%, respectively showed trend like difference in the detection frequency between the ABO blood groups. RDA analysis showed a trend like clustering (p = 0.06) of the ABO blood groups (Figure 28). Statistically significant difference in the diversity of the *Bifidobacterium* PCR-DGGE profiles was detected between AB and O blood groups (p=0.04), O group having the highest and AB the lowest diversity (Figure 29).

**Table 15**

| Statistically significant differences in the band presence between ABO blood group samples as determined by *Bifidobacterium* group-specific DGGE. (N=64 samples with blood groups A = 21 samples, B = 13, AB = 11 and O = 19). Statistical testing, Fisher's exact test was based on band presence/absence. | | | |
|---|---|---|---|
| **Genotype (band position)** | **p-value** | **number of hits** | **Distribution according to ABO blood group** |
| 24.9% | 0.06 | 2 | A (0%); B (17%); O (0%); AB (0%) |
| 26.6% | 0.007 | 40 | A (63%); B (58%); O (94%); AB (60%) |
| 53.5% | 0.07 | 50 | A (100%); B (75%); O (88%); AB (80%) |

### Example 16

### Associations between occurrence of DGGE genotypes and ABO blood group of the host

Occurrence of several DGGE-derived band position genotypes were significantly associated with the presence of a specific ABO blood group antigen in the host (Table 16).

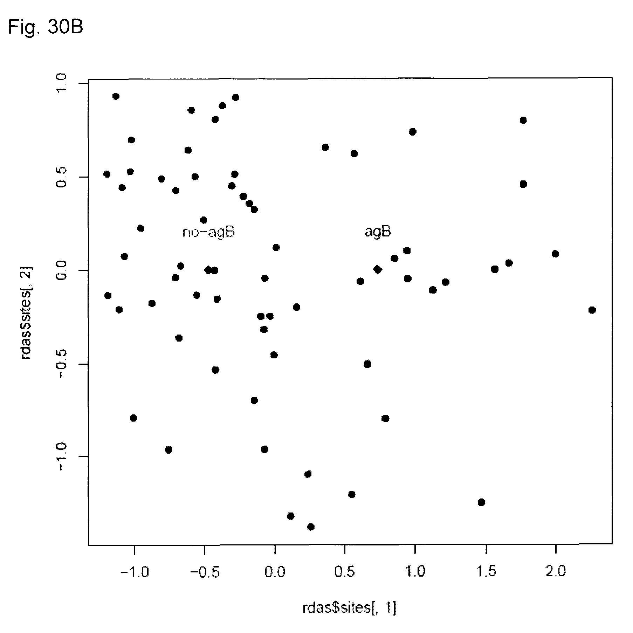

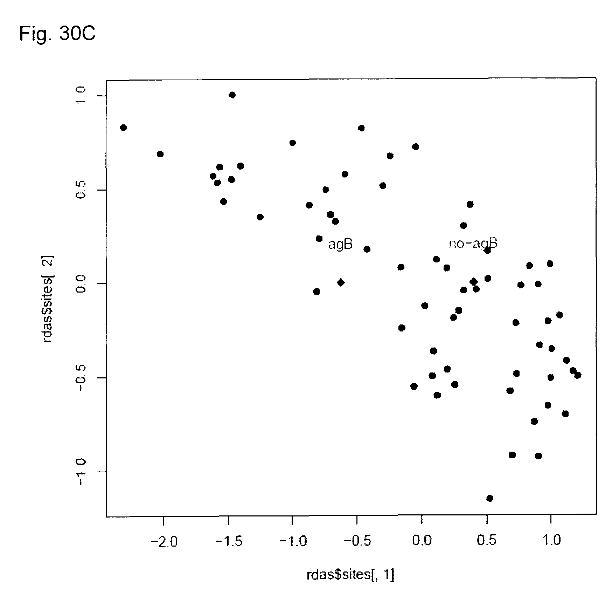

Blood group antigen based RDA-comparisons of PCR-DGGE-data revealed three PCR-DGGE primer-sets, where B-antigen containing blood groups (B and AB) and non-B-antigen containing blood groups (A and O) clustered with statistically significant difference (Figure 30). The p-values were: Universal primers (0.005), C. *leptum* (0.01) and EREC (0.005).

**Table 16**

| Association of the occurrence of bacterial genotypes with A, B and O blood group antigens in the host. Statistical testing, Fisher's exact text was based on band presence/absence. | | | |
|---|---|---|---|
| **DGGE genotype, number of genotypes** | **B+AB vs. O+A (p-value)** | **A+AB vs. O+B** | **O vs. A+AB+B** |
| UNIV, 18.00%, n=9 | 35% vs 3% (0.002) | 6% vs. 22% | 5% vs. 35% |
| UNIV, 31.40%, n=21 | 48% vs. 23% (0.014) | 38% vs. 28% | 42% vs. 11% |
| UNIV, 32.20%, n=8 | 30% vs. 3% (0.004) | 13% vs. 13% | 5% vs. 16% |
| UNIV, 33.80%, n=56 | 74% vs. 95% (0.004) | 84% vs. 91% | 100% vs. 82% |
| UNIV, 39.00%, n=9 | 17% vs. 13% | 25% vs. 3% (0.026) | 5% vs. 18% |
| UNIV, 42.20%, n=9 | 30% vs. 5% (0.022) | 16% vs. 13% | 0% vs. 20% |
| UNIV, 47.00%, n=7 | 22% vs. 5% (0.012) | 9% vs. 13% | 5% vs. 13% |
| UNIV, 49.40%, n=8 | 0% vs. 20% (0.018) | 13% vs. 13% | 21% vs. 9% |
| UNIV, 58.80%, n=11 | 30% vs. 8% (0.002) | 16% vs. 19% | 11% vs. 20% |
| UNIV, 61.10%, n=17 | 17% vs. 0% (0.020) | 9% vs. 3% | 0% vs. 9% |
| Lacto, 9.00%, n=11 | 16% vs. 10% (0.092) | 16% vs. 19% | 11% vs. 20% |
| Lacto, 14.10%, n=15 | 26% vs. 18% | 25% vs. 22% | 5% vs. 31% (0.028) |
| Lacto, 15.40%, n=5 | 17% vs. 3 (0.072) | 9% vs. 6% | 0 vs. 11% |
| Lacto 66.30%, n=10 | 17% vs. 15% | 25% vs. 6% (0.082) | 5% vs. 20% |
| Lacto, 74.20%, n=3 | 0% vs. 8% | 0% vs. 9% | 6% vs. 0% (0.023) |
| Lacto, 83.10%, n=4 | 9% vs. 0% | 0% vs. 6% | 0% vs. 4% |
| Lacto, 84.70%, n=40 | 65% vs. 59% | 59% vs. 66% | 74% vs. 58% |
| Lacto, 86.60%, n=3 | 0% vs. 8% | 0% vs. 9% | 16% vs. 0% (0.023) |
| Lacto, 92.30%, n=8 | 4% vs. 18% | 6% vs. 19% | 32% vs. 4% (0.007) |
| EREC 4.8%, n=13 | 22% vs. 20% | 34% vs. 6% (0.011) | 5% vs. 27% |
| EREC 35.30%, n=8 | 26% vs. 5% (0.048) | 16% vs. 9% | 5% vs. 16% |
| EREC, 39.70%, n=9 | 26% vs. 5% (0.022) | 16% vs. 13% | 0% vs. 20% (0.048) |
| EREC, 46.90%, n=19 | 52% vs. 18% (0.004) | 31 % vs. 28% | 11 % vs. 38% (0.004) |
| EREC, 50.90%, n=34 | 70% vs. 43% (0.021) | 53% vs. 53% | 37% vs. 60% |
| EREC, 61.10%, n=18 | 43% vs. 20% (0.044) | 22% vs. 34% | 32% vs. 27% |
| EREC, 73.90%, n=28 | 61% vs. 35% (0.043) | 44% vs. 44% | 37% vs. 47% |
| C. *leptum,* 11.90%, n=31 | 22% vs. 63% (0.002) | 47% vs. 50% | 63% vs. 42% |
| C. *leptum,* 15.40%, n=8 | 22% vs. 8% (0.048) | 6% vs. 19% | 5% vs. 16% |
| C. *leptum,* 16.00%, n=6 | 26% vs. 0% (0.002) | 16% vs. 3% | 0% vs. 13% |
| C. *leptum,* 20.50%, n=9 | 26% vs.8% (0.022) | 13% vs. 16% | 5% vs. 18% |
| C. *leptum,* 38.80%, n=8 | 22% vs. 8% (0.048) | 16% vs. 8% | 0% vs. 18% |
| C. *leptum,* 52.10%, n=8 | 4% vs. 18% | 9% vs. 16% | 26% vs. 7% (0.044) |
| C. *leptum,* 67.90%, n=12 | 30% vs. 13% (0.048) | 13% vs. 25% | 11% vs. 22% |
| C. *leptum,* 84.00%, n=7 | 0% vs. 18% (0.037) | 6% vs. 16% | 26% vs. 4% (0.021) |
| Bfra, 5.00%, n=5 | 21 % vs. 0% (0.008) | 6% vs. 9% | 0% vs. 11% |
| Bfra, 9.90%, n=10 | 21% vs. 13% | 26% vs. 6% (0.043) | 5% vs. 20% |
| Bfra, 21.50%, n=9 | 25% vs. 10% (0.023) | 6% vs. 22% | 11 % vs. 16% |
| Bfra, 36.80%, n=7 | 0% vs. 18% (0.036) | 10% vs. 13% | 21% vs. 7% |
| Bfra, 62.80%, n=5 | 0% vs. 13% | 3% vs. 13% | 21% vs. 2% (0.026) |
| Bifido, 26.60%, n=40 | 59% vs. 77% | 62% vs. 79% | 94% vs. 61% (0.022) |

### Example 17

### Identification of DNA sequences encoding the 16S rRNA for each DGGE genotype

Each band position in DGGE gel (= DNA fragment) was excised and the DNA fragment was sequenced. In addition, strains isolated from the faecal samples of the individuals were analysed in DGGE in order to screen strains with 16S rRNA gene fragment melting behaviour (i.e. sequence) identical to the observed DGGE bands of faecal samples. Tables 17 and 18 summarizes the DNA sequences fpound for each DGGE genotype.

**Table 17**

| Identification by DNA sequencing the 16S rRNA gene of lactobacilli representing the band positions showing a ABO blood group related difference in the *Lactobacillus* DGGE. The identifications were based on the sequence analysis of bands extracted from the DGGE gel (14.10%) or by analysing faecal isolates and *Lactobacillus* spp. type strains in the DGGE gel followed by the comparison of the band positions of the isolates/strains with the corresponding band positions detected for the faecal samples. | |
|---|---|
| **Band position** | **16S rRNA gene fragment defining the DGGE band position** |
| 9.00% | |
| 14.1% | |
| 15.4% | not available |
| 66.3% | |
| 74.2%* | |
| 83.1% | |
| 84.7%. | |
| 86.6% | |
| 92.3% | |
| | |

| | |
|---|---|
| * DGGE genotype 74.2% defined by this sequence of 16S rRNA gene fragment; the genotype 74.20% always co-occurred with genotype 66.30%. | |

**Table 18**

| Identification by DNA sequencing the 16S rRNA gene of bifidobacteria representing the band positions showing a ABO blood group related difference in the *Bifidobacterium* DGGE. The identifications were based on the sequence analysis of bands extracted from the DGGE gel or by analysing faecal isolates and *Bifidobacterium* spp. type strains in the DGGE gel followed by the comparison of the band positions of the isolates/strains with the corresponding band positions detected for the faecal samples. | |
|---|---|
| **Band position** | **16S rRNA gene fragment defining the DGGE band position** |
| 24.9% | not available |
| 26.6% | |
| 53.5% | |

### Example 18

### Adhesion of microbes onto ABO molecules

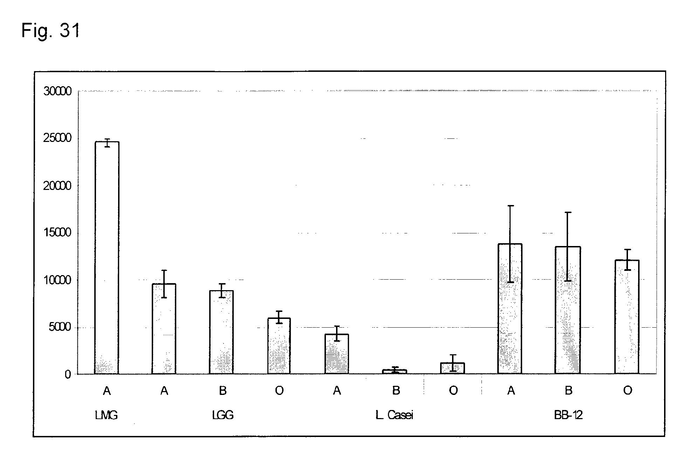

ABO adhesion tests were performed on several public culture collection/commercial strains. The data presented in Figure 31 clearly showed differences in adhesion efficiency between different strains, and also when the results were compared with a known high A-antigen adherer *L. crispatus* LMG 18199. Columns present averaged data of strain adhesions on A-, B- and O-antigens with ±SD of several measurements: *L. crispatus* LMG 18199 40 repetitions, *L. rhamnosus* LGG 8 repetitions and other strains 4 repetitions. All statistical comparisons between *L. crispatus* LMG 18199 and other tested strains had p-values below 0.02.

## Claims

1. A method of tailoring a bacterial composition for an individual having certain ABO blood group genotype based on the spectrum of bacteria found in the mucosal tissue of at least one individual with the same ABO blood group genotype, comprising:
determining the ABO blood type of the recipient of the bacterial composition;
determining the typical mucosal bacterial repertoire of the specific ABO blood type by determining which bacterial strains are more common in the intestine of individuals having:
(a) B or AB blood group genotypes than individuals having A or O blood group genotypes;
(b) A or AB blood group genotypes than individuals having B or O blood group genotypes; and/or
(c) A, B or AB blood group genotypes than individuals having O blood group genotype; and
manufacturing the bacterial composition based on the determined bacterial repertoire.

2. The method according to claim 1, **characterized in that** the ABO blood group genotype is A or B or O.

3. The method according to claim 1, **characterized in that** the ABO blood group genotype is AB.

4. The method according to any one of claims 1 to 3 **characterized in that** the bacterial composition is tailored for an individual who has received hematopoietic stem cell graft or who has celiac disease.

## Patentansprüche

1. Verfahren zum Zuschneiden einer bakteriellen Zusammensetzung auf ein Individuum mit einem bestimmten ABO-Blutgruppengenotyp anhand des Spektrums von im Schleimhautgewebe wenigstens eines Individuums mit dem gleichen ABO-Blut-gruppengenotyp angetroffenen Bakterien, umfassend:
Bestimmen der ABO-Blutgruppe des Empfängers der bakteriellen Zusammensetzung;
Bestimmen des typischen Bakterienrepertoires der Schleimhaut für die spezifische AB0-Blut-gruppe, indem bestimmt wird, welche Bakterienstämme häufiger im Darm von Individuen mit:
(a) B- oder AB-Blutgruppengenotypen als von Individuen mit A- oder 0-Blutgruppengenotypen vorkommen;
(b) A- oder AB-Blutgruppengenotypen als von Individuen mit B- oder 0-Blutgruppengenotypen vorkommen; und/oder
(c) A-, B- oder AB-Blutgruppengenotypen als von Individuen mit 0-Blutgruppengenotyp vorkommen; und
Herstellen der bakteriellen Zusammensetzung anhand des bestimmten Bakterienrepertoires.

2. Verfahren nach Anspruch 1, **dadurch gekennzeichnet, dass** es sich bei dem AB0-Blutgruppengenotyp um A oder B oder 0 handelt.

3. Verfahren nach Anspruch 1, **dadurch gekennzeichnet, dass** es sich bei dem AB0-Blutgruppengenotyp um AB handelt.

4. Verfahren nach einem der Ansprüche 1 bis 3, **dadurch gekennzeichnet, dass** die bakterielle Zusammensetzung auf ein Individuum zugeschnitten wird, das ein Transplantat von hämatopoetischen Stammzellen erhalten hat oder das an Zöliakie leidet.

## Revendications

1. Procédé de production d'une composition bactérienne sur mesure pour un individu présentant un certain génotype de groupe sanguin ABO, sur la base du spectre de bactéries se trouvant dans le tissu muqueux d'au moins un individu présentant le même génotype de groupe sanguin ABO, comprenant :
déterminer le groupe sanguin ABO du destinataire de la composition bactérienne ;
déterminer le répertoire bactérien muqueux typique du groupe sanguin ABO spécifique en déterminant les souches bactériennes qui sont plus courantes dans l'intestin d'individus présentant :
(a) les génotypes de groupe sanguin B ou AB que dans celui d'individus présentant les génotypes de groupe sanguin A ou 0 ;
(b) les génotypes de groupe sanguin A ou AB que dans celui d'individus présentant les génotypes de groupe sanguin B ou 0 ; et/ou
(c) les génotypes de groupe sanguin A, B ou AB que dans celui d'individus présentant le génotype de groupe sanguin 0 ; et
fabriquer la composition bactérienne sur la base du répertoire bactérien déterminé.

2. Procédé selon la revendication 1, **caractérisé en ce que** le génotype de groupe sanguin ABO est A ou B ou O.

3. Procédé selon la revendication 1, **caractérisé en ce que** le génotype de groupe sanguin ABO est AB.

4. Procédé selon l'une quelconque des revendications 1 à 3, **caractérisé en ce que** la composition bactérienne est produite sur mesure pour un individu ayant reçu une greffe de cellules souches hématopoïétiques ou souffrant de la maladie coeliaque.
